# EUROPEAN PATENT SPECIFICATION

(11) **EP 1 009 817 B1**
(45) Date of publication and mention of the grant of the patent: **23.09.2009**
(21) Application number: 98935654.8
(22) Date of filing: 14.07.1998
(51) Int. Cl.: C12N 15/00, C07K 14/705, C07K 16/28, C07K 19/00, A61K 38/00, A01K 67/027

(54) **RTD RECEPTOR**
RTD RECEPTOR
RECEPTEUR RTD

(30) Priority: 26.08.1997 US 918874
(43) Date of publication of application: 21.06.2000
(73) Proprietor: Genentech, Inc., South San Francisco CA 94080-4990 (US)
(72) Inventor: ASHKENAZI, Avi, J., San Mateo, CA 94401 (US); GURNEY, Austin, Belmont, CA 94002 (US)
(74) Representative: Kiddle, Simon John
(86) International application number: PCT/US1998/014552
(87) International publication number: WO 1999/010484

(56) References cited:
- US-A- 5 447 851
- DEGLI ESPOSITI M.A. ET AL.: "The novel receptor TRAIL-R4 induces NF-kappaB and protects against TRAIL-mediated apoptosis, yet retains an incomplete death domain" IMMUNITY, vol. 7, no. 6, 7 December 1997, pages 813-820, XP002087442 US
- MARSTERS S.A. ET AL.: "A novel receptor for Apo2L/TRAIL contains a truncated death domain" CURRENT BIOLOGY, vol. 7, no. 12, December 1997, pages 1003-1006, XP002087443 GB
- PAN G. ET AL.: "TRUDD, a new member of the trail receptor family that antagonizes TRAIL signalling" FEBS LETTERS, vol. 424, no. 1-2, 6 March 1998, pages 41-45, XP002087444 AMSTERDAM NL
- SCHNEIDER P ET AL: "CHARACTERIZATION OF TWO RECEPTORS FOR TRAIL" FEBS LETTERS, vol. 416, 27 October 1997, pages 329-334, XP002065022
- MACFARLAND M ET AL: "IDENTIFICATION AND MOLECULAR CLONING OF TWO NOVEL RECEPTORS FOR THE CYTOTOXIC LIGAND TRAIL" JOURNAL OF BIOLOGICAL CHEMISTRY, vol. 272, no. 41, 10 October 1997, pages 25417-25420, XP002065148
- SHERIDAN J P ET AL: "CONTROL OF TRAIL-INDUCED APOPTOSIS BY A FAMILY OF SIGNALING AND DECOY RECEPTORS" SCIENCE, vol. 277, 8 August 1997, pages 818-821, XP002065023
- PAN G ET AL: "AN ANTAGONIST DECOY RECEPTOR AND A DEATH DOMAIN-CONTAINING RECEPTOR FOR TRAIL" SCIENCE, vol. 277, 8 August 1997, pages 815-818, XP002065147

## Description

### FIELD OF THE INVENTION

The present invention relates generally to the identification, isolation, and recombinant production of novel polypeptides, designated herein as "RTD" and to anti-RTD antibodies.

### BACKGROUND OF THE INVENTION

### Apoptosis or "Programmed Cell Death"

Control of cell numbers in mammals is believed to be determined, in part, by a balance between cell proliferation and cell death. One form of cell death, sometimes referred to as necrotic cell death, is typically characterized as a pathologic form of cell death resulting from some trauma or cellular injury. In contrast, there is another, "physiologic" form of cell death which usually proceeds in an orderly or controlled manner. This orderly or controlled form of cell death is often referred to as "apoptosis" (see, *e.g*., Barr et al., Bio/Technology, 12:487-493 (1994); Steller et al., Science, 267: 1443-1449 (1995)]. Apoptotic cell death naturally occurs in many physiological processes, including embryonic development and clonal selection in the immune system [Itoh et al:, Cell, 66:233-243 (199))]. Decreased levels of apoptotic cell death have been associated with a variety of pathological conditions, including cancer, lupus, and herpes virus infection [Thompson, Science, 267: 1456-1462(1995)]. Increased levels of apoptotic cell death may be associated with a variety of other pathological conditions, including AIDS, Alzheimers disease, Parkinson's disease, amyotrophic lateral sclerosis, multiple sclerosis, retinitis pigmentosa, cerebellar degeneration, aplastic anemia, myocardial infarction, stroke, reperfusion injury, and toxin-induced liver disease [see, Thompson, supra].

Apoptotic cell death is typically accompanied by one or more characteristic morphological and biochemical changes in cells, such as condensation of cytoplasm, loss of plasma membrane microvilli, segmentation of the nucleus, degradation of chromosomal DNA or loss of mitochondrial function. A variety of extrinsic and intrinsic signals are believed to trigger or induce such morphological and biochemical cellular changes [Raff, Nature, 356:397-400 (1992); Steller, supra; Sachs et al., Blood, 82:15 (1993)]. For instance, they can be triggered by hormonal stimuli, such as glucocorticoidhormones for immature thymocytes, as well as withdrawal of certain growth factors [Watanabe-Fukunagaet al., Nature, 356:314:317 (1992)]. Also, some identified oncogenes such as *myc, rel.* and *EIA,* and tumor suppressors, like *p53*, have been reported to have a role in inducing apoptosis. Certain chemotherapy drugs and some forms of radiation have likewise been observed to have apoptosis-inducing activity [Thompson, supra].

### TNF Family of Cytokines

Various molecules, such as tumor necrosis factor-α ("TNF-α"), tumor necrosis factor-β ("TNF-β" or "lymphotoxin"), CD30 ligand, CD27 ligand, CD40 ligand, OX-40 ligand, 4-1 BB ligand, Apo-1 ligand (also referred to as Fas ligand or CD95 ligand), and Apo-2 ligand (also referred to as TRAIL) have been identified as members of the tumor necrosis factor ("TNF") family of cytokines [See, e.g., Gruss and Dower, Blood, 85:3378-3404(1995); Wiley et al., Immunity, 3 :673-682 (1995); Pitti et al., J. Biol. Chem., 271:12687-12690 (1996)]. Among these molecules, TNF-α, TNF-β, CD30 ligand, 4-IBB ligand, Apo-I ligand, and Apo-2 ligand (TRAIL) have been reported to be involved in apoptotic cell death. Both TNF-α and TNF-β have been reported to induce apoptotic death in susceptible tumor cells [Schmid et al., Proc. Natl. Acad. Sci., 83:1881 (1986); Dealtry et al., Eur. J. Immunol., 17:689 (1987)]. Zheng et al. have reported that TNF-α is involved in post-stimulation apoptosis of CD8-positive T cells [Zheng et al., Nature. 377:348-351 (1995)]. Other investigators have reported that CD30 ligand may be involved in deletion of self-reactive T cells in the thymus (Amakawa et al., Cold Spring Harbor Laboratory Symposium on Programmed Cell Death, Abstr. No. 10, (1995)].

Mutations in the mouse Fas/Apo-I receptor or ligand genes (called *lpr* and *gld*, respectively) have been associated with some autoimmunedisorders, indicating that Apo- I ligand may play a role in regulating the clonal deletion of self reactive lymphocytes in the periphery [Krammeret al., Curr. Op. Immunol., 6:279-289 (1994); Nagata et al., Science, 267:1449-1456 (1995)]. Apo-I ligand is also reported to induce post-stimulation apoptosis in CD4-positive T lymphocytes and in B lymphocytes, and may be involved in the elimination of activated lymphocytes when their function is no longer needed [Krammer et al., supra; Nagata et al., supra]. Agonist mouse monoclonal antibodies specifically binding to the Apo-1 receptor have been reported to exhibit cell killing activity that is comparable to or similar to that of TNF-α [Yonehara et al., L Exp. Med., 169:1747-1756 (1989)].

### TNF Family of Receptors

Induction of various cellular responses mediated by such TNF family cytokines is believed to be initiated by their binding to specific cell receptors. Two distinct TNF receptors of approximately 55-kDa. (TNFR1) and 75-kDa (TNFR2) have been identified[Hohman et al., J. Biol. Chem., 264:14927-14934(1989); Brockhaus et al., Proc. Natl. Acad. Sci., 87:3127-3131 (1990); EP 417,563, published March 20, 1991] and human and mouse cDNAs corresponding to both receptor types have been isolated and characterized [Loetscheret al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026(1991)]. Extensive polymorphisms have been associated with both TNF receptor genes [see, e.g., Takao et al., Immunogenetics, 37:199-203 (1993)]. Both TNFRs share the typical structure of cell surface receptors including extracellular, transmembrane and intracellular regions. The extracellular portions of both receptors are found naturally also as soluble TNF-binding proteins [Nophar, Y. et al., EMBO J., 9:3269 (1990); and Kohno, T. et al., Proc. Natl. Acad. Sci. U.S.A., 87:8331 (1990)]. More recently, the cloning of recombinant soluble TNF receptors was reported by Hale et al. [J. Cell. Biochem. Supplement 15F, 1991, p. 113 (P424)].

The extracellular portion of type I and type 2 TNFRs (TNFR1 and TNFR2) contains a repetitive amino acid sequence pattern of four cysteine-rich domains (CRDs) designated I through 4, starting from the NH₂-terminus. Each CRD is about 40 amino acids long and contains 4 to 6 cysteine residues at positions which are well conserved [Schall et al., supra; Loetscheret al., supra; Smith et al., supra; Nophar et al., supra; Kohno et al., supra] In TNFR1, the approximate boundaries of the four CRDs are as follows: CRD1- amino acids 14 to about 53; CRD2- amino acids from about 54 to about 97; CRD3- amino acids from about 98 to about 138; CRD4- amino acids from about 139 to about 167. In TNFR2, CRD1 includes amino acids 17 to about 54; CRD2-amino acids from about 55 to about 97; CRD3-amino acids from about 98 to about 140; and CRD4- amino acids from about 141 to about 179 [Banner et al., Cell, 73:431-435 (1993)]. The potential role of the CRDs in ligand binding is also described by Banner et al., supra.

A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 (Mallet et al., EMBO J., 9: 1063 (1990)] and the Fas antigen [Yonehara et al., supra and Itoh et al., supra]. CRDs are also found in the soluble TNFR (sTNFR)-like T2 proteins of the Shope and myxoma poxviruses [Upton et al., Virology, 160:20-29 (1987); Smith et al., Biochem. Biophys. Res. Commun., 176:335 (1991); Upton et al., Virology, 184:370 (1991)]. Optimal alignment of these sequences indicates that the positions of the cysteine residues are well conserved. These receptors are sometimes collectively referred to as members of the TNF/NGF receptor superfamily. Recent studies on p75NGFR showed that the deletion of CRD1 (Welcher, A.A. et al., Proc. Natl. Acad. Sci. USA, 88: 159-163 (1991)] or a 5-amino acid insertion in this domain [Yan, H. and Chao, M.V., J. Biol. Chem., 266:12099-12104 (1991)] had little or no effect on NGF binding [Yan, H. and Chao, M. V., supra.] p75 NG FR contains a proline-rich stretch of about 60 amino acids, between its CRD4 and transmembrane region, which is not involved in NGF binding [Peetre, C. et al., Eur. J. Hematol., 41:414-419 (1988); Seckinger, P. et al., J. Biol. Chem., 264:11966-11973 (1989); Yan, H. and Chao, M.V., supra]. A similar proline-rich region is found in TNFR2 but not in TNFR1.

Itoh et al. disclose that the Apo-1 receptor can signal an apoptotic cell death similar to that signaled by the 55-kDa TNFR1 [Itoh et al., supra]. Expression of the Apo-I antigen has also been reported to be down-regulated along with that of TNFR1 when cells are treated with either TNF-α or anti-Apo-1 mouse monoclonal antibody [Krammer et al., supra; Nagata et al., supra]. Accordingly, some investigators have hypothesized that cell lines that co-express both Apo-I and TNFR1 receptors may mediate cell killing through common signaling pathways [Id].

The TNF family ligands identified to date, with the exception of lymphotoxin-α, are type II transmembrane proteins, whose C-terminus is extracellular. In contrast, the receptors in the TNF receptor (TNFR) family identified to date are type I transmembrane proteins. In both the TNF ligand and receptor families, however, homology identified between family members has been found mainly in the extracellular domain ("ECD"). Several of the TNF family cytokines, including TNF-α, Apo-I ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

Recently, other members of the TNFR family have been identified. In Marsters et al., Curr. Biol. 6:750 (1996), investigators described a full length native sequence human polypeptide, called Apo-3, which exhibits similarity to the TNFR family in its extracellular cysteine-rich repeats and resembles TNFR1 and CD95 in that it contains a cytoplasmic death domain sequence [see also Marsters et al., Curr. Biol., 6:1669 (1996)]. Apo-3 has also been referred to by other investigators as DR3, ws1-1 and TRAMP [Chinnaiyan et al., Science, 274:990 (1996); Kitson et al., Nature, 384:372 (1996): Bodmer et al., Immunity, 6:79 (1997)].

Pan et al. have disclosed another TNF receptor family member referred to as "DR4" [Pan et al., Science, 276:111-113 (1997)]. The DR4 was reported to contain a cytoplasmic death domain capable of engaging the cell suicide apparatus. Pan et al. disclose that DR4 is believed to be a receptor for the ligand known as Apo-2 ligand or TRAIL.

In Sheridan et al., Science, 277:818-821(1997) and Pan et al., Science, 277: 815-818 (1997), another molecule believed to be a receptor for the Apo-2 ligand (TRAIL) is described. That molecule is referred to as DR5 (it has also been alternatively referred to as Apo-2). Like DR4, DR5 is reported to contain a cytoplasmic death domain and be capable of signaling apoptosis.

In Sheridan et al., supra, a receptor called DcR1 (or alternatively, Apo-2DcR) is disclosed as being a potential decoy receptor for Apo-2 ligand (TRAIL). Sheridan et al. report that **DeRI** can inhibit Apo-2 ligand function *in vitro.* See also, Pan et al., supra, for disclosure on the decoy receptor referred to as TRID.

### The Apoptosis-Inducing Signaling Complex

As presently understood, the cell death program contains at least three important elements - activators, inhibitors, and effectors; in *C*. *elegans,* these elements are encoded respectively by three genes, *Ced-4, Ced-9* and *Ced-3* [Steller, Science, 267:1445 (1995); Chinnaiyan et al., Science, 275:1122-1126(1997); Wang et al., Cell, 90:1-20 (1997)]. Two of the TNFR family members, TNFR1 and Fas/ApoI (CD95), can activate apoptotic cell death [Chinnaiyan and Dixit, Current Biology, 6:555-562 (1996); Fraser and Evan, Cell; 85:781-784 (1996)]. TNFR1 is also known to mediate activation of the transcription factor, NF-κB [Tartaglia et al., Cell, 74:845-853 (1993); Hsu et al., Cell, 84:299.308 (1996)]. In addition to some ECD homology, these two receptors share homology in their intracellular domain (ICD) in an oligomerization interface known as the death domain [Tartaglia et al., supra; Nagata, Cell, 88:355(1997)]. Death domains are also found in several metazoan proteins that regulate apoptosis, namely, the Drosophila protein, Reaper, and the mammalian proteins referred to as FADD/MORTI, TRADD, and RIP [Cleaveland and Ihle, Cell, 81:479-482 (1995)). Using the yeast-two hybrid system, Raven et al. report the identification of protein, wsl-1, which binds to the TNFR1 death domain [Raven et al., Programmed Cell Death Meeting, September 20-24, 1995, Abstract at page 127; Raven et al., European Cytokine Network, 7:Abstr. 82 at page 210 (April-June 1996); see also, Kitson et al., Nature, 384:372-375 (1996)]. The ws1-1 protein is described as being homologous to TNFR1 (48% identity) and having a restricted tissue distribution. According to Raven et al.; the tissue distribution of wsl-1 is significantly different from the TNFR1 binding protein, TRADD.

Upon ligand binding and receptor clustering, TNFR 1 and CD95 are believed to recruit FADD into a death-inducing signalling complex. CD95 purportedly binds FADD directly, while TNFR1 binds FADD indirectly via TRADD [Chinnaiyan et al., Cell, 81:505-512 (1995); Boldin et al., J. Biol. Chem., 270:387-391 (1995); Hsu et al., supra; Chinnaiyan et al., J. Biol. Chem., 271:4961-4965 (1996)]. It has been reported that FADD serves as an adaptor protein which recruits the *Ced-3*-related protease, MACHα/FLICE (caspase 8), into the death signalling complex [Boldin et al., Cell, 85:803-815 (1996); Muzio et al., Cell, 85:817-827 (1996)]. MACHα/FLICE appears to be the trigger that sets off a cascade of apoptotic proteases, including the interleukin-1β converting enzyme (ICE) and CPP32/Yama, which may execute some critical aspects of the cell death programme (Fraser and Evan, supra].

It was recently disclosed that programmed cell death involves the activity of members of a family ofcysteineproteasesrelatedto the *C. elegans* cell death gene, *ced-3*, and to the mammalian IL-1-converting enzyme, ICE. The activity of the ICE and CPP32/Yama proteases can be inhibited by the product of the cowpox virus gene, *crmA* [Ray et al., Cell, 69:597-604 (1992); Tewari et al., Cell, 81:801-809 (1995)]. Recent studies show that CnnA can inhibit TNFP1- and CD95-induced cell death (Enarl et al., Nature. 375:78-81 (1995); Tewari et al., J. Biol. CHem., 70:3255-3260 (1995)].

As reviewed recently by Tewari et al., TNFR1, TNFR2 and CD40 modulate the expression of proinflammatory and costimulatory cytokines, cytokine receptors, and cell adhesion molecules through activation of the transcription factor, NF-κB (Tewari et al., Curr. Op. Genet. Develop., 6:39-44 (1996)]. NF-κB is the prototype of a family of dimeric transcription factors whose subunits contain conserved Rel regions [Verma et al., Genes Develop., 9:2723-2735 (1996); Baldwin, Ann. Rev. Immunol., 14:649-681 (1996)]. In its latent form, NF-κB is complexed with members of the IκB inhibitor family; upon inactivation of the IκB in response to certain stimuli, released NF-κB translocates to the nucleus where it binds to specific DNA sequences and activates gene transcription.

For a review of the TNF family of cytokines and their receptors, see Gruss and Dower, supra

### SUMMARY OF THE INVENTION

Applicants have identified cDNA clones that encode novel polypeptides, designated in the present application as "RTD." It is believed that RTD is a member of the TNFR family; full-length native sequence human RTD polypeptide exhibits similarity to the TNFR family in its extracellular cysteine-rich repeats. Applicants found that RTD can bind Apo-2 ligand (Apo-2L) and block Apo-2L induced apoptosis. It is presently believed that RTD may function as an inhibitory Apo-2L receptor.

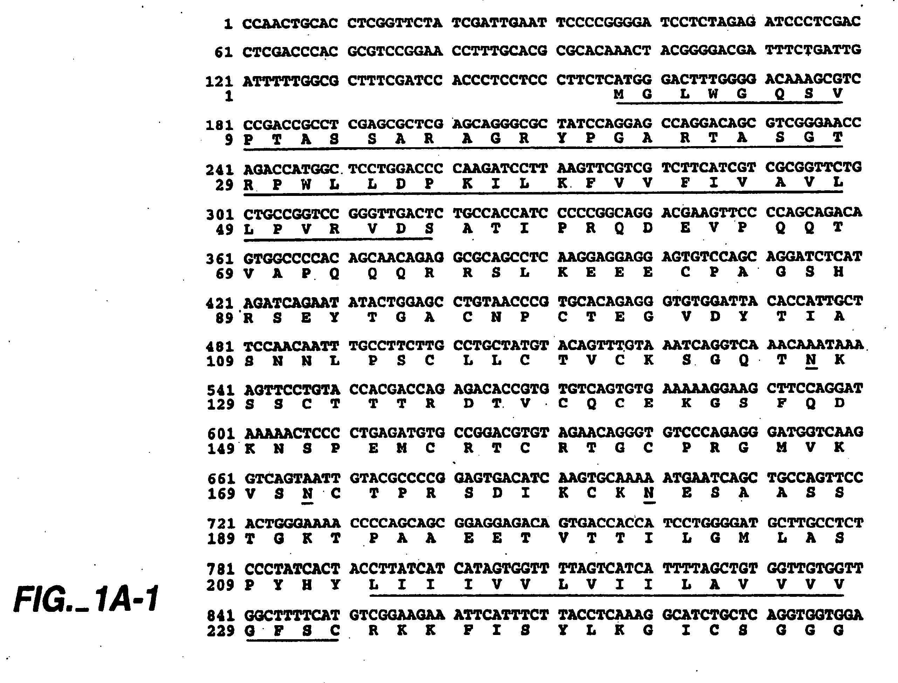

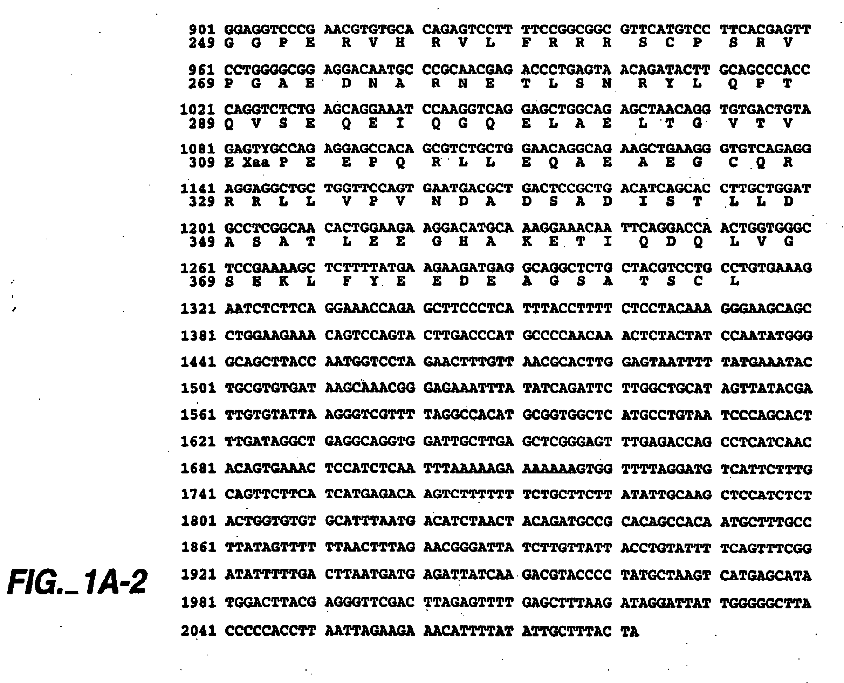

In one embodiment, the invention provides isolated RTD polypeptide. In particular, the invention provides isolated native sequence RTD polypeptide, which in one embodiment, includes an amino acid sequence comprising residues 1 to 386 of Figure 1A (SEQ ID NO:1). In other embodiments, the isolated RTD polypeptide comprises at least 95% amino acid sequence identity with native sequence RTD polypeptide comprising residues 1 to 386 of Figure 1A (SEQ ID NO:1). The isolated RTD polypeptide may also comprise a polypeptide which lacks a signal sequence. Optionally, such polypeptide may comprise residues 56 to 386 of Figure 1A (SEQ ID NO:1).

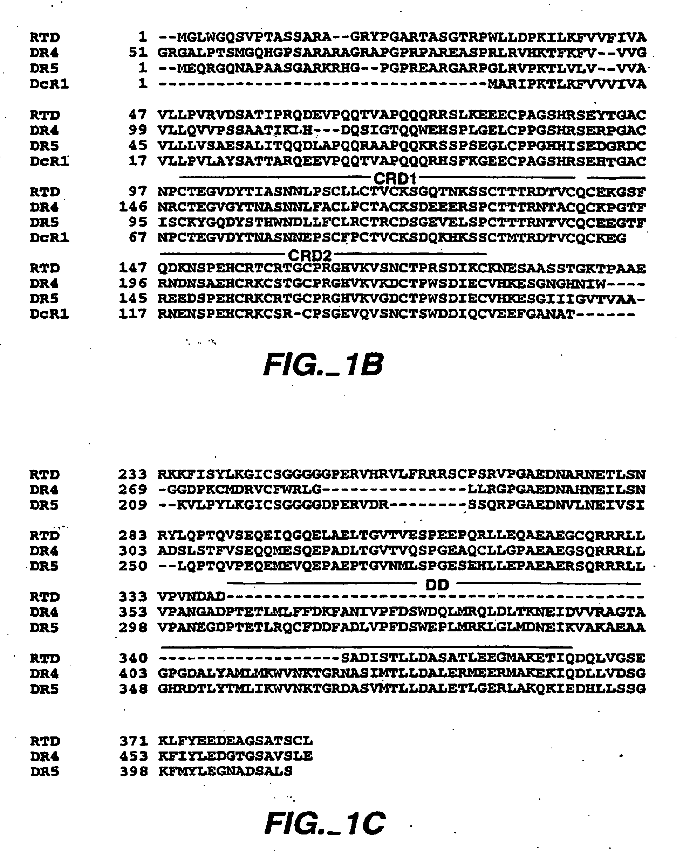

In another embodiment, the invention provides an isolated extracellular domain (ECD) sequence of RTD. Optionally, the isolated extracellular domain sequence comprises amino acid residues 56 to 212 of Fig. 1A (SEQ ID NO: 1). The isolated RTD ECD polypeptide may also comprise a polypeptide containing one or more cysteine rich domains. In one such embodiment, the polypeptide comprises one or both cysteine rich domains identified in Figure 1B as residues 99 to 139 and 141 to 180, respectively, of SEQ ID NO:1.

In another embodiment, the invention provides chimeric molecules comprising RTD polypeptide fused to a heterologous polypeptide or amino acid sequence. An example of such a chimeric molecule comprises a RTD fused to an immunoglobulin sequence. Another example comprises an extracellular domain sequence of RTD fused to a heterologous polypeptide or amino acid sequence, such as an immunoglobulin sequence.

In another embodiment, the invention provides an isolated nucleic acid molecule encoding RTD polypeptide. In one aspect, the nucleic acid molecule is RNA or DNA that encodes a RTD polypeptide or a particular domain of RTD, or is complementary to such encoding nucleic acid sequence, and remains stably bound to it under at least moderate, and optionally, under high stringency conditions. In one embodiment, the nucleic acid sequence is selected from:
(a) the coding region of the nucleic acid sequence of Figure 1A (SEQ ID NO:2) that codes for residue 1 to residue 386 (i.e., nucleotides 157-159 through 1312-1314), inclusive;
(b) the coding region of the nucleic acid sequence of Figure 1A (SEQ ID NO:2) that codes for residue 56 to residue 212 (i.e., nucleotides 321-323 through 789-791), inclusive; or
(c) a sequence corresponding to the sequence of (a) or (b) within the scope of degeneracy of the genetic code.

In a further embodiment, the invention provides a vector comprising the nucleic acid molecule encoding the RTD polypeptide or particular domain of RTD. A host cell comprising the vector or the nucleic acid molecule is also provided. A method of producing RTD is further provided.

In another embodiment, the invention provides an antibody which specifically binds to RTD. The antibody may be an agonistic, antagonistic or neutralizing antibody.

In another embodiment, the invention provides non-human, transgenic or knock-out animals.

A further embodiment of the invention provides articles of manufacture and kits that include RTD or RTD antibodies.

### BRIEF DESCRIPTION OF THE DRAWINGS

Figure 1A shows the nucleotide sequence of a native sequence human RTD cDNA and its derived amino acid sequence. In Figure 1A, the signal sequence (residues 1-55) and the transmembrane sequence (residues 213-232) are underlined. The potential N-linked glycosylation sites (residues 127, 171, and 182) are also underlined.
Figure 1B shows the deduced amino acid sequence of human RTD ECD aligned with corresponding ECDs of DR4, DR5, and DcR1. The cysteine rich domains are identified as CRD I and CRD2.
Figure 1C shows the deduced amino acid sequence of the human RTD intracellular region aligned with corresponding intracellular regions of DR4 and DR5. The death domain is identified as DD.
Figure 1D is a schematic diagram of the putative domain organization of RTD, DR4, DR5, and DcR1 and showing the extracellular region [including the signal (S) and cysteine rich domains (CRD1 and CRD2)], transmembrane (TM) and truncated death domain (TD) or death domain (DD). In DcR1, 1-5 indicate 15 amino acid pseudorepeats.
Figure 2A shows binding of radioiodinated Apo-2L to purified RTD ECD immunoadhesin as measured in a co-precipitation assay.
Figure 2B shows inhibition of Apo-2L induction of apoptosis by RTD ECD immunoadhesin in cultured HeLa cells.
Figure 3A shows apoptosis induction in HeLa cells transfected with DR4 or DR5; HeLa cells transfected with full-length RTD (clone DNA35663 or clone DNA35664) did not result in any difference in apoptosis as compared to control transfected cells.
Figure 3B shows the results of an electrophoretic mobility shift assay testing for NF-κB activation. 293 cells were transfected with vector alone, RTD (clone DNA35663 or clone DNA35664) or DR4 or DR5. RTD transfection did not result in an increase in NF-κB activity.
Figure 3C shows blocking of Apo-2 ligand induced apoptosis in 293 cells transfected with RTD (clone DNA35663 or clone DNA35664).
Figure 4 shows expression of RTD mRNA in human tissues as analyzed by Northern blot hybridization. The sizes of molecular weight standards are shown on the right in kb.

### DETAILED DESCRIPTION OF THE PREFERRED EMBODIMENTS

### 1. Definitions

The terms "RTD polypeptide" and "RTD," when used herein encompass native sequence RTD and RTD variants (which are further defined herein). These terms encompass RTD from a variety of mammals, including humans. The RTD may be isolated from a variety of sources, such as from human tissue types or from another source, or prepared by recombinant or synthetic methods.

A "native sequence RTD" comprises polypeptide having the same amino acid sequence as an RTD derived from nature. Thus, a native sequence RTD can have the amino acid sequence of naturally-occurring RTD from any mammal. Such native sequence RTD can be isolated from nature or can be produced by recombinant or synthetic means. The term "native sequence RTD" specifically encompasses naturally-occurring truncated or secreted forms of the RTD (*e.g*., an extracellular domain sequence), naturally-occurring variant forms (*e.g*., alternatively spliced forms) and naturally-occurring allelic variants of the RTD. A naturally-occurring variant form of the RTD includes a RTD having an amino acid substitution shown in Fig. 1A (SEQ ID NO-1). In one embodiment of such naturally-occurring variant form, the serine residue at position 310 is substituted by a leucine residue. In Fig. 1A (SEQ ID NO:1), the amino acid residue at position 310 is identified as "Xaa" to indicate that the amino acid may, optionally, be either serine or leucine. In Fig. 1A (SEQ ID NO:2), the nucleotide at position 1085 is identified as "Y" to indicate that the nucleotide may be either cytosine(C) or thymine (T) or uracil (U). In one embodiment of the invention, the native sequence RTD is a mature or full-length native sequence RTD comprising amino acids 1 to 386 of Fig. 1A (SEQ ID NO:1). Optionally, the RTD is one which lacks a signal sequence, and may comprise residues 56 to 386 of Figure 1A (SEQ ID NO:1).

The "RTD extracellular domain" or "RTD ECD" refers to a form of RTD which is essentially free of transmembrane and cytoplasmic domains. Ordinarily, RTD ECD will have less than 1% of such transmembrane and cytoplasmic domains and preferably, will have less than 0.5% of such domains. Optionally, RTD ECD will comprise amino acid residues 56 to 212 of Fig. 1A (SEQ ID NO:1). The RTD ECD may also comprise a polypeptide containing one or more cysteine rich domains, and may comprise a polypeptide which includes one or both cysteine rich domains identified as residues 99 to 139 and 141 to 180, respectively, of Figure 1A (SEQ ID NO:1). The invention further provides fragments of such soluble RTD ECD molecules. Preferably, the ECD fragments retain the biological activity and/or properties of the full length RTD or the ECD identified herein as having amino acid residues 56 to 212 of Figure 1A (SEQ ID NO:1).

"RTD variant" means a biologically active RTD as defined below having at least 95% amino acid sequence identity with the RTD having the deduced amino acid sequence shown in Fig. 1A (SEQ ID NO:1) for a full-length native sequence human RTD. Such RTD variants include, for instance, RTD polypeptides wherein one or more amino acid residues are added, or deleted, at the N- or C-terminus of the sequence of Fig. 1A (SEQ ID NO:1).

"Percent (%) amino acid sequence identity" with respect to the RTD sequences identified herein is defined as the percentage of amino acid residues in a candidate sequence that are identical with the amino acid residues in the RTD sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as ALIGN or Megalign (DNASTAR) software. Those skilled in the art can determine appropriate parameters for measuring alignment, including any algorithms needed to achieve maximal alignment over the full length of the sequences being compared.

The term "epitope tagged" when used herein refers to a chimeric polypeptide comprising RTD, or a domain sequence thereof, fused to a "tag polypeptide". The tag polypeptide has enough residues to provide an epitope against which an antibody can be made, yet is short enough such that it does not interfere with activity of the RTD. The tag polypeptide preferably also is fairly unique so that the antibody does not substantially cross-react with other epitopes. Suitable tag polypeptides generally have at least six amino acid residues and usually between about 8 to about 50 amino acid residues (preferably, between about 10 to about 20 residues).

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the RTD natural environment will not be present. Ordinarily, however, isolated polypeptide will be prepared by at least one purification step.

An "isolated" RTD nucleic acid molecule is a nucleic acid molecule that is identified and separated from at least one contaminant nucleic acid molecule with which it is ordinarily associated in the natural source of the RTD nucleic acid. An isolated RTD nucleic acid molecule is other than in the form or setting in which it is found in nature. Isolated RTD nucleic acid molecules therefore are distinguished from the RTD nucleic acid molecule as it exists in natural cells. However, an isolated RTD nucleic acid molecule includes RTD nucleic acid molecules contained in cells that ordinarily express RTD where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadestsense and specifically covers single anti-RTD monoclonal antibodies (including agonist, antagonist, and neutralizing antibodies) and anti-RTD antibody compositions with polyepitopic specificity.

The term "monoclonal antibody" as used herein refers to an antibody obtained from a population of substantially homogeneous antibodies, *i.e.*, the individual antibodies comprising the population are identical except for possible naturally-occurring mutations that may be present in minor amounts. Monoclonal antibodies are highly specific, being directed against a single antigenic site. Furthermore, in contrast to conventional(polyclonal)antibody preparations which typically include different antibodies directed against different determinants (epitopes), each monoclonal antibody is directed against a single determinant on the antigen.

The monoclonal antibodies herein include hybrid and recombinant antibodies produced by splicing a variable (including hypervariable) domain of an anti-RTD antibody with a constant domain (*e.g.* "humanized" antibodies), or a light chain with a heavy chain, or a chain from one species with a chain from another species, or fusions with heterologous proteins, regardless of species of origin or immunoglobulinclass or subclass designation, as well as antibody fragments (*e.g.*, Fab, F(ab')₂, and Fv), so long as they exhibit the desired biological activity. See, *e.g.* U.S. Pat. No. 4,816,567 and Mage et al., in Monoclonal Antibody Production Techniques and Applications, pp.79-97 (Marcel Dekker, Inc.: New York, 1987).

Thus, the modifier "monoclonal" indicates the character of the antibody as being obtained from a substantially homogeneous population of antibodies,and is not to be construed as requiring production of the antibody by any particular method. For example, the monoclonal antibodies to be used in accordance with the present invention may be made by the hybridoma method first described by Kohler and Milstein, Nature, 256:495 (1975), or may be made by recombinant DNA methods such as described in U.S. Pat. No. 4,816,567. The "monoclonal antibodies" may also be isolated from phage libraries generated using the techniques described in McCafferty et al., Nature, 348:552-554 (1990), for example.

"Humanized" forms of non-human(*e.g.* murine) antibodies are specific chimeric immunoglobulins, immunoglobulin chains, or fragments thereof (such as Fv, Fab, Fab', F(ab')₂ or other antigen-binding subsequencesof antibodies)which contain minimal sequence derived from non-human immunoglobulin. For the most part, humanized antibodies are human immunoglobulins(recipientantibody) in which residues from a complementary determining region (CDR) of the recipient are replaced by residues from a CDR of a non-human species (donor antibody) such as mouse, rat, or rabbit having the desired specificity, affinity, and capacity. In some instances, Fv framework region (FR) residues of the human immunoglobulin are replaced by corresponding non-human residues. Furthermore, the humanized antibody may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulinconsensus sequence. The humanized antibody optimally also will comprise at least a portion of an immunoglobulin constant region or domain (Fc), typically that of a human immunoglobulin.

"Biologicallyactive" and "desired biological activity" for the purposes herein means (1) having the ability to modulate apoptosis (either in an agonistic or stimulating manner or in an antagonistic or blocking manner) in at least one type of mammalian cell *in vivo* or *ex vivo*; (2) having the ability to bind Apo-2 ligand: or (3) having the ability to modulate the activity of Apo-2 ligand.

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays, FACS analysis or DNA electrophoresis, all of which are known in the art.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, blastoma, gastrointestinal cancer, renal cancer, pancreatic cancer, glioblastoma, neuroblastoma, cervical cancer, ovarian cancer, liver cancer, stomach cancer, bladder cancer, hepatoma, breast cancer, colon cancer, colorectal cancer, endometrial cancer, salivary gland cancer, kidney cancer, prostate cancer, vulval cancer, thyroid cancer, hepatic carcinoma, and various types of head and neck cancer.

The terms "treating," "treatment," and "therapy" as used herein refer to curative therapy, prophylactic therapy, and preventative therapy.

The term "mammal" as used herein refers to any mammal classified as a mammal, including humans, cows, horses, dogs and cats. In a preferred embodiment of the invention, the mammal is a human.

### II. Compositions and Methods of the Invention

The present invention provides newly identified and isolated RTD polypeptides. In particular, Applicants have identified and isolated various human RTD polypeptides. The properties and characteristics of some of these RTD polypeptides are described in further detail in the Examples below. Based upon the properties and characteristics of the RTD polypeptides disclosed herein, it is Applicants' present belief that RTD is a member of the TNFR family, and particularly, is a receptor for Apo-2 ligand.

A description follows as to how RTD, as well as RTD chimeric molecules and anti-RTD antibodies, may be prepared.

### A. Preparation of RTD

The description below relates primarily to production of RTD by culturing cells transformed or transfected with a vector containing RTD nucleic acid. It is of course, contemplated that alternative methods, which are well known in the art, may be employed to prepare RTD.

### 1. Isolation of DNA Encoding RTD

The DNA encoding RTD may be obtained from any cDNA library prepared from tissue believedto possess the RTD mRNA and to express it at a detectable level. Accordingly, human RTD DNA can be conveniently obtained from a cDNA library prepared from human tissues, such as libraries of human cDNA described in Example 1. The RTD-encoding gene may also be obtained from a genomic library or by oligonucleotide synthesis.

Libraries can be screened with probes (such as antibodies to the RTD or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding RTD is to use PCR methodology [Sambrook et al., supra; Dieffenbach et al ; PCR Primer: A laboratory Manual (Cold Spring Harbor Laboratory Press, 1995)].

One method of screeningemploys selected oligonucleotide sequences to screen cDNA libraries from various human tissues. Example I below describes techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra

Nucleic acid having all the protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

RTD variants can be prepared by introducing appropriate nucleotide changes into the RTD DNA, or by synthesis of the desired RTD polypeptide. Those skilled in the art will appreciate that amino acid changes may alter post-translational processes of the RTD, such as changing the number or position of glycosylation sites or altering the membrane anchoring characteristics.

Variations in the native full-length sequence RTD or in various domains of the RTD described herein, can be made, for example, using any of the techniques and guidelines for conservative and non-conservative mutations set forth, for instance, in U.S. Pat. No. 5,364,934. Variations may be a substitution, deletion or insertion of one or more codons encoding the RTD that results in a change in the amino acid sequence of the RTD as compared with the native sequence RTD. Optionally the variation is by substitution of at least one amino acid with any other amino acid in one or more of the domains of the RTD molecule. The variations can be made using methods known in the an such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the RTD variant DNA.

Scanning amino acid analysis can also be employed to identify one or more amino acids along a contiguous sequence which are involved in the interaction with a particular ligand or receptor. Among the preferred scanning amino acids are relatively small, neutral amino acids. Such amino acids include alanine, glycine, serine, and cysteine. Alanine is the preferred scanning amino acid among this group because it eliminates the side-chain beyond the beta-carbon and is less likely to alter the main-chain conformation of the variant. Alanine is also preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

Once selected RTD variants are produced, they can be contacted with, for instance, Apo-2L, and the interaction, if any, can be determined. The interaction between the RTD variant and Apo-2L can be measured by an *in vitro* assay, such as described in the Examples below. While any number of analytical measurements can be used to compare activities and properties between a native sequence RTD and a RTD variant, a convenient one for binding is the dissociation constant K_{d} of the complex formed between the RTD variant and Apo-2L as compared to the K_{d} for the native sequence RTD. Generally, a ≥ 3-fold increase or decrease in K_{d} per substituted residue indicates that the substituted residue(s) is active in the interaction of the native sequence RTD with the Apo-2L. Selected RTD variants may also be analyzed for biological activity, such as the ability to modulate apoptosis, in the *in vitro* assays described in the Examples.

Optionally, representative sites in the RTD sequence suitable for mutagenesis would include sites within the extracellular domain, and particularly, within one or more of the cysteine-rich domains. Such variationscan be accomplished using the methods described above. Deletional variants of the ECD, such as fragments resulting from the deletion of one or more amino acids, are encompassed by the invention. Preferably, such deletional variants or fragments retain at least one biological activity or property of the full length or soluble forms of RTD.

### 2. Insertion of Nucleic Acid into A Replicable Vector

The nucleic acid (*e.g.*, cDNA or genomic DNA) encoding RTD may be inserted into a replicable vector for further cloning (amplification of the DNA) or for expression. Various vectors are publicly available. The vector components generally include, but are not limited to, one or more of the following: a signal sequence, an origin of replication, one or more marker genes, an enhancer element, a promoter, and a transcription termination sequence, each of which is described below.

### (i) Signal Sequence Component

The RTD may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the RTD DNA that is inserted into the vector. The heterologous signal sequence selected preferably is one that is recognized and processed (*i.e.*, cleaved by a signal peptidase) by the host cell. The signal sequence may be a prokaryotic signal sequence selected, for example, from the group of the alkaline phosphatase, penicillinase, lpp, or heat-stable enterotoxin II leaders. For yeast secretion the signal sequence may be, *e.g.*, the yeast invertase leader, alpha factor leader (including *Saccharomyces* and *Kluyveromyces* α-factor leaders, the latter described in U.S. Pat. No. 5,010,182), or acid phosphatase leader, the *C. albicans* glucoamylase leader (EP 362,179 published 4 April 1990), or the signal described in WO 90/13646 published 15 November 1990. In mammalian cell expression the native RTD presequence that normally directs insertion of RTD in the cell membrane of human cells *in vivo* is satisfactory, although other mammalian signal sequences may be used to direct secretion of the protein, such as signal sequences from secreted polypeptides of the same or related species, as well as viral secretory leaders, for example, the herpes simplex glycoprotein D signal.

The DNA for such precursor region is preferably ligated in reading frame to DNA encoding RTD.

### (ii) Origin of Replication Component

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors(the SV40 origin may typically be used because it contains the early promoter).

Most expression vectors are "shuttle" vectors, *i.e.*, they are capable of replication in at least one class of organisms but can be transfected into another organism for expression. For example, a vector is cloned in *E. coli* and then the same vector is transfected into yeast or mammalian cells for expression even though it is not capable of replicating independently of the host cell chromosome.

DNA may also be amplified by insertion into the host genome. This is readily accomplished using *Bacillus species* as hosts, for example, by including in the vector a DNA sequence that is complementary to a sequence found in *Bacillus* genomic DNA. Transfectionof *Bacillus* with this vector results in homologous recombination with the genome and insertion of RTD DNA. However, the recovery of genomic DNA encoding RTD is more complex than that of an exogenously replicated vector because restriction enzyme digestion is required to excise the RTD DNA.

### (iii) Selection Gene Component

Expression and cloning vectors typically contain a selection gene, also termed a selectable marker. This gene encodes a protein necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, *e.g.*, ampicillin, neomycin, methotrexate, or tetracycline, (b) complement auxotrophic deficiencies, or (c) supply critical nutrients not available from complex media, *e.g.*, the gene encoding D-alanine racemase for *Bacilli.*

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin [Southern et al., J. Molec. Appl. Genet., 1:327 (1982)], mycophenolic acid (Mulligan et al., Science, 209:1422 (1980)] or hygromycin [Sugden et al., Mol. Cell. Biol., 5:410-413 (1985)]. The three examples given above employ bacterial genes under eukaryotic control to convey resistance to the appropriate drug G418 or neomycin (geneticin), xgpt (mycophenolic acid), or hygromycin, respectively.

Another example of suitable selectable markers for mammalian cells are those that enable the identification of cells competent to take up the RTD nucleic acid, such as DHFR or thymidine kinase. The mammalian cell transformants are placed under selection pressure that only the transformants are uniquely adapted to survive by virtue of having taken up the marker. Selection pressure is imposed by culturing the transformants under conditions in which the concentration of selection agent in the medium is successively changed, thereby leading to amplification of both the selection gene and the DNA that encodes RTD. Amplification is the process by which genes in greater demand for the production of a protein critical for growth are reiterated in tandem within the chromosomes of successive generations of recombinant cells. Increased quantities of RTD are synthesized from the amplified DNA. Other examples of amplifiable genes include metallothionein-I and -11, adenosine deaminase, and ornithine decarboxylase.

Cells transformed with the DHFR selection gene may first be identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity, prepared and propagated as described by Urlaub et al., Proc. Natl. Acad. Sci. USA, 77:4216 (1980). The transformed cells are then exposed to increased levels of methotrexate. This leads to the synthesis of multiple copies of the DHFR gene, and, concomitantly, multiple copies of other DNA comprising the expression vectors, such as the DNA encoding RTD. This amplificationtechnique can be used with any otherwise suitable host, *e.g.*, ATCC No. CCL61 CHO-K1, notwithstanding the presence of endogenous DHFR if, for example, a mutant DHFR gene that is highly resistant to Mtx is employed (EP 117,060).

Alternatively, host cells (particularly wild-type hosts that contain endogenous DHFR) transformed or co-transformed with DNA sequences encoding RTD, wild-type DHFR protein, and another selectable marker such as aminoglycoside 3'-phosphotransferase (APH) can be selected by cell growth in medium containing a selection agent for the selectable marker such as an aminoglycosidic antibiotic, *e.g.*, kanamycin, neomycin, or G418. See U.S. Patent No. 4,965,199.

A suitable selection gene for use in yeast is the *trp*I gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*I gene provides a selection marker for a mutant strain of yeast lacking the ability to grow in tryptophan, for example, ATCC No. 44076 or PEP4-1 [Jones, Genetics, 85:12 (1977)]. The presence of the *trp*1 lesion in the yeast host cell genome then provides an effective environment for detecting transformation by growth in the absence of tryptophan. Similarly, Leu2-deficient yeast strains (ATCC 20,622 or 38,626) are complemented by known plasmids bearing the *Leu*2 gene.

In addition, vectors derived from the 1.6 µm circular plasmid pKD1 can be used for transformation of *Kluyveromyces* yeasts [Bianchi et al., Curr. Genet., 12:185 (1987)]. More recently, an expression system for large-scale production of recombinant calf chymosin was reported for *K. lactis* [Van den Berg, Bio/Technology, 8:135 (1990)]. Stable multi-copy expression vectors for secretion of mature recombinant human serum albumin by industrial strains of *Kluyveromyces* have also been disclosed [Fleer et al., Bio/Technology, 9:968-975 (1991)].

### (iv) Promoter Component

Expression and cloning vectors usually contain a promoter that is recognized by the host organism and is operably linked to the RTD nucleic acid sequence. Promoters are untranslated sequences located upstream (5') to the start codon of a structural gene (generally within about 100 to 1000 bp) that control the transcription and translation of particular nucleic acid sequence, such as the RTD nucleic acid sequence, to which they are operably linked. Such promoters typically fall into two classes, inducible and constitutive. Inducible promoters are promoters that initiate increased levels of transcription from DNA under their control in response to some change in culture conditions, *e.g.*, the presence or absence of a nutrient or a change in temperature. At this time a large number of promoters recognized by a variety of potential host cells are well known. These promoters are operably linked to RTD encoding DNA by removing the promoter from the source DNA by restriction enzyme digestion and inserting the isolated promoter sequence into the vector. Both the native RTD promoter sequence and many heterologous promoters may be used to direct amplification and/or expression of the RTD DNA.

Promoters suitable for use with prokaryotic hosts include the β-lactamase and lactose promoter systems [Chang et al., Nature, 275:615 (1978); Goeddel et al., Nature, 281:544(1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. However, other known bacterial promoters are suitable. Their nucleotide sequences have been published, thereby enabling a skilled worker operably to ligate them to DNA encoding RTD [Siebenlist et al., Cell, 20:269 (1980)] using linkers or adaptors to supply any required restriction sites. Promoters for use in bacterial systems also will contain a Shine-Dalgamo (S.D.) sequence operably linked to the DNA encoding RTD.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CXCAAT region where X may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochromeC, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

RTD transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters,*e.g.*, the actin promoter or an immunoglobulin promoter, from heat-shock promoters, and from the promoter normally associated with the RTD sequence, provided such promoters are compatible with the host cell systems.

The early and late promoters of the SV40 virus are conveniently obtained as an SV40 restriction fragmentthat also contains the SV40 viral origin of replication [Fiers et al.. Nature, 273: 113 (1978); Mulligan and Berg, Science; 209(1422-1427(1980); Pavlakis et al., Proc. Natl. Acad. Sci. USA, 78:7398-7402 (1981)]. The immediate early promoter of the human cytomegalovirus is conveniently obtained as a HindIII E restriction fragment [Greenaway et al., Gene, 18:355-360 (1982)]. A system for expressing DNA in mammalian hosts using the bovine papilloma virus as a vector is disclosed in U.S. Patent No. 4,419,446. A modificationof this system is described in U.S. Patent No. 4,601,978 [See also Gray et al., Nature, 295:503-508 (1982) on expressing cDNA encoding immune interferon in monkey cells; Reyes et al., Nature, 297:598-601 (1982) on expression of human β-interferon cDNA in mouse cells under the control of a thymidine kinase promoter from herpes simplex virus; Canaan i and Berg, Proc. Natl. Acad. Sci. USA 79:5166-5170 (1982) on expression of the human interferon β1 gene in cultured mouse and rabbit cells; and Gorman et al., Proc. Natl. Acad. Sci. USA, 79:6777-6781 (1982) on expression of bacterial CAT sequences in CV-1 monkey kidney cells, chicken embryo fibroblasts, Chinese hamster ovary cells, HeLa cells, and mouse NIH-3T3 cells using the Rous sarcoma virus long terminal repeat as a promoter].

### (v) Enhancer Element Component

Transcription of a DNA encoding the RTD of this invention by higher eukaryotes may be increased by inserting an enhancer sequence into the vector. Enhancers are cis-acting elements of DNA, usually about from 10 to 300 bp, that act on a promoterto increase its transcription. Enhancers are relatively orientation and position independent, having been found 5' [Laimins et al., Proc. Natl. Acad, Sci. USA, 78:993 (1981]) and 3' [Lusky et al., Mol. Cell Bio., 3:1108 (1983]) to the transcription unit, within an intron [Banerji et al., Cell, 33:729 (1983)], as well as within the coding sequence itself [Osborne et al., Mol. Cell Bio., 4:1293 (1984)]. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature, 297:17-18 (1982) on enhancing elements for activation of eukaryotic promoters. The enhancer may be spliced into the vector at a position 5' or 3' to the RTD coding sequence, but is preferably located at a site 5' from the promoter.

### (vi) Transcription Termination Component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding RTD.

### (vii) Construction and Analysis of Vectors

Construction of suitable vectors containing one or more of the above-listed components employs standard ligation techniques. Isolated plasmids or DNA fragments are cleaved, tailored, and re-ligated in the form desired to generate the plasmids required.

For analysis to confirm correct sequences in plasm ids constructed, the ligation mixtures can be used to transform *E. coli* K12 strain 294 (ATCC 31,446) and successful transformants selected by ampicillin or tetracycline resistance where appropriate. Plasmids from the transformants are prepared, analyzed by restriction endonuclease digestion, and/or sequenced by the method of Messing et al., Nucleic Acids Res., 9:309 (1981) or by the method of Maxam et al., Methods in Enzymology, 65:499 (1980).

### (viii) Transient Expression Vectors

Expression vectors that provide for the transient expression in mammalian cells of DNA encoding RTD may be employed. In general, transient expression involves the use of an expression vector that is able to replicate efficiently in a host cell, such that the host cell accumulates many copies of the expression vector and, in turn, synthesizes high levels of a desired polypeptide encoded by the expression vector [Sambrook et al., *supra*]*.* Transient expression systems, comprising a suitable expression vector and a host cell, allow for the convenientpositive identification of polypeptides encoded by cloned DNAs, as well as for the rapid screening of such polypeptides for desired biological or physiological properties. Thus, transient expression systems are particularly useful in the invention for purposes of identifying RTD variants.

### (ix) Suitable Exemplary Vertebrate Cell Vectors

Other methods, vectors, and host cells suitable for adaptation to the synthesis of RTD in recombinant vertebrate cell culture are described in Gething et al., Nature, 293:620-625 (1981); Mantei et al., Nature, 281:40-46 (1979); EP 117,060; and EP 117,058.

### 3. Selection and Transformation of Host Cells

Suitable host cells for cloning or expressing the DNA in the vectors herein are the prokaryote, yeast, or higher eukaryote cells described above. Suitable prokaryotes for this purpose include but are not limited to eubacteria, such as Gram-negative or Gram-positive organisms, for example, Enterobacteriaceaesuch as *Escherichia, e.g., E. coli, Enterobacter, Erwinia, Klebsiella, Proteus, Salmonella, e.g., Salmonella typhimurium, Serratia. e.g., Serratia marcescans,* and *Shigella,* as well as *Bacilli* such as *B. subtilis* and *B. licheniformis* (*e.g., B. licheniformis* 41 P disclosed in DD 266,710 published 12 April 1989), *Pseudomonas* such as *P. aeruginosa,* and *Streptomyces.* Preferably, the host cell should secrete minimal amounts of proteolytic enzymes.

In addition to prokaryotes, eukaryotic microbes such as filamentous fungi or yeast are suitable cloning or expression hosts for RTD-encoding vectors. *Saccharomyces cerevisiae,* or common baker's yeast, is the most commonly used among lower eukaryotic host microorganisms. However, a number of other genera, species, and strains are commonly available and useful herein.

Suitable host cells for the expression of glycosylated RTD are derived from multicellular organisms. Such host cells are capable of complex processing and glycosylation activities. In principle, any higher eukaryotic cell culture is workable, whether from vertebrate or invertebrate culture. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito)*, Drosophila melanogaster* (fruitfly), and *Bombyx mori* have been identified [See, *e.g*., Luckow et al., Bio/Technology, 6:47-55 (1988); Miller et al., in Genetic Engineering, Setlow et al., eds., Vol. 8 (Plenum Publishing,1986), pp. 277-279; and Maeda et al., Nature, 315:92-594 (1985)]. A variety of viral strains for transfection are publicly available, *e.g*., the L-1 variant of *Autographacalifornica* NPV and the Bm-5 strain of *Bombyx mori* NPV.

Plant cell cultures of cotton, com, potato, soybean, petunia, tomato, and tobacco can be utilized as hosts. Typically, plant cells are transfected by incubation with certain strains of the bacterium *Agrobacterium tumefaciens.* During incubation of the plant cell culture with *A*. *tumefaciens,* the DNA encoding the RTD can be transferred to the plant cell host such that it is transfected, and will, under appropriate conditions, express the RTD-encoding DNA. In addition, regulatory and signal sequences compatible with plant cells are available, such as the nopaline synthase promoter and polyadenylation signal sequences [Depicker et al.,J Mol.Appl.Gen., 1:561 (1982)]. In addition, DNA segments isolated from the upstream region of the T-DNA *780* gene are capable of activating or increasing transcription levels of plant-expressible genes in recombinant DNA-containing plant tissue [EP 321,196 published 21 June 1989].

Propagation of vertebrate cells in culture (tissue culture) is also well known in the art [See, *e.g*., Tissue Culture, Academic Press, Kruse and Patterson, editors (1973)]. Examples of useful mammalian host cell lines are monkey kidney CV line transformed by SV40 (COS-7, ATCC CRL 1651); human embryonic kidney line (293 or 293 cells subcloned for growth in suspension culture, Graham et al., J. Gen Virol.; 36:59 (1977)); baby hamster kidney cells (BHK, ATCC CCL 10); Chinese hamster ovary cells/-DHFR(CHO, Urlaub and Chasin, Proc. Natl. Acad. Sci. USA, 77:4216 (1980)); mouse sertoli cells (TM4, Mather, Biol. Reprod., 23:243.251 (1980)); monkey kidney cells (CV I ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 (1982)); MRC 5 cells; and FS4 cells.

Host cells are transfected and preferably transformed with the above-describedexpression or cloning vectors for RTD production and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

Transformation means introducingDNA into an organism so that the DNA is replicable, either as an extrachromosomal element or by chromosomal integrant. Depending on the host cell used, transformation is done using standard techniques appropriate to such cells. The calcium treatment employing calcium chloride, as described in Sambrooket al., supra, or electroporation is generally used for prokaryotes or other cells that contain substantial cell-wall barriers. Infection with *Agrobacterium tumefaciens* is used for transformation of certain plant cells, as described by Shaw et al., Gene. 23:315 (1983) and WO 89/05859 published 29 June 1989. In addition, plants may be transfected using ultrasound treatment as described in WO 91/00358 published 10 January 1991.

For mammalian cells without such cell walls, the calcium phosphate precipitation method of Graham and van der Eb, Virology, 52:456457 (1978) is preferred. General aspects of mammalian cell host system transformations have been described in U.S. Pat. No. 4,399,216. Transformations into yeast are typically carried out according to the method of Van Solingen et al., J. Bact., 130:946 (1977) and Hsiao et al., Proc. Natl. Acad. Sci. (USA), 76:3829(1979). However, other methods for introducing DNA into cells, such as by nuclear microinjection, electroporation, bacterial protoplast fusion with intact cells, or polycations, *e.g*., polybrene, polyornithine, may also be used. For various techniques for transforming mammalian cells, see Keown et al., Methods in Enzymology, 185:27-537 (1990) and Mansour et al., Nature, 336:348-352 (1988).

### 4. Culturing the Host Cells

Prokaryotic cells used to produce RTD may be cultured in suitable media as described generally in Sambrook et al., supra.

The mammalian host cells used to produce RTD may be cultured in a variety of media. Examples ofcommerciallyavailable media include Ham's F10 (Sigma), Minimal Essential Medium ("MEM", Sigma), RPMI-1640 (Sigma), and Dulbecco's Modified Eagle's Medium ("DMEM", Sigma). Any such media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin^{™} drug), trace element (defined as inorganic compounds usually present at final concentrations in the micromolar range); and glucose or an equivalent energy source. Any other necessary supplements may also be included at appropriate concentrations that would be known to those skilled in the art. The culture conditions, such as temperature, pH, and the like; are those previously used with the host cell selected for expression, and will be apparent to the ordinarily skilled artisan.

In general, principles, protocols, and practical techniques for maximizing the productivity of mammalian cell cultures can be found in Mammalian Cell Biotechnology: a Practical Approach, M. Butler, ed. (IRL Press, 1991).

The host cells referred to in this disclosure encompass cells in culture as well as cells that are within a host animal.

### 5. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201 -5205 (1980)], dot blotting (DNA analysis), or in situ hybridization, using an appropriately labeled probe, based on the sequences provided herein. Various labels may be employed, most commonly radioisotopes, and particularly ³²P. However, other techniques may also be employed, such as using biotin-modified nucleotides for introduction into polynucleotide. The biotin then serves as the site for binding to avidin or antibodies, which may be labeled with a wide variety of labels, such as radionucleotides, fluorescers or enzymes. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemicalstaining of cells or tissue sections and assay of cell culture or body fluids, to quantitate directly the expression of gene product. With immunohistochemical staining techniques, a cell sample is prepared, typically by dehydration and fixation, followed by reaction with labeled antibodies specific for the gene product coupled, where the labels are usually visually, detectable, such as enzymatic labels, fluorescent labels, or luminescent labels.

Antibodies useful for immunohistochemical staining and/or assay of sample fluids may be either monoclonal or polyclonal, and may be prepared in any mammal. Conveniently, the antibodies may be prepared against a native sequence RTD polypeptide or against a synthetic peptide based on the DNA sequences provided herein or against exogenous sequence fused to RTD DNA and encoding a specific antibody epitope.

### 6. Purification of RTD Polypeptide

Forms of RTD may be recovered from culture medium or from host cell lysates. If the RTD is membrane-bound, it can be released from the membrane using a suitable detergent solution (*e.g*. Triton-X 100) or its extracellulardomain may be released by enzymatic cleavage. RTD can also be released from the cell-surface by enzymatic cleavage of its glycophospholipid membrane anchor.

When RTD is produced in a recombinant cell other than one of human origin, the RTD is free of proteins or polypeptidesof human origin. However, it may be desired to purify RTD from recombinant cell proteins or polypeptides to obtain preparations that are substantially homogeneous as to RTD. As a first step, the culture medium or lysate may be centrifuged to remove particulate cell debris. RTD thereafter is purified from contaminant soluble proteins and polypeptides, with the following procedures being exemplary of suitable purification procedures: by fractionation on an ion-exchange column; ethanol precipitation; reverse phase HPLC; chromatographyon silica or on a cation-exchangeresin such as DEAE; chromatofocusing;SDS-PAGE; ammonium sulfate precipitation; gel filtration using, for example, Sephadex G-75; and protein A Sepharose columns to remove contaminants such as IgG.

RTD variants in which residues have been deleted, inserted, or substituted can be recovered in the ' same fashion as native sequence RTD, taking account of changes in properties occasioned by the variation. For example, preparation of an RTD fusion with another protein or polypeptide, *e.g*., a bacterial or viral antigen, immunoglobulin sequence, or receptor sequence, may facilitate purification; an immunoaffinity column containing antibody to the sequence can be used to adsorb the fusion polypeptide. Other types of affinity matrices also can be used.

A protease inhibitor such as phenyl methyl sulfonyl fluoride (PMSF) also may be useful to inhibit proteolytic degradation during purification, and antibiotics may be included to prevent the growth of adventitious contaminants. One skilled in the art will appreciate that purification methods suitable for native sequence RTD may require modification to account for changes in the character of RTD or its variants upon expression in recombinant cell culture.

### 7. Covalent Modifications of RTD Polypeptides

Covalent modifications of RTD are included within the scope of this invention. One type of covalent modification of the RTD is introduced into the molecule by reacting targeted amino acid residues of the RTD with an organic derivatizing agent that is capable of reacting with selected side chains or the Nor C- terminal residues of the RTD.

Derivatization with bifunctional agents is useful for crosslinking RTD to a water-insoluble support matrix or surface for use in the method for purifying anti-RTD antibodies, and vice-versa. Derivatizationwith one or more bifunctional agents will also be useful for crosslinking RTD molecules to generate RTD dimers. Such dimers may increase binding avidity and extend half-life of the molecule *in vivo.* Commonly used cross linking agents include, *e.g*., 1,1-bis(diazoacetyl)-2-phenylethaneglutaraldehyde,N-hydroxysuccinimide esters, for example, esters with 4-azidosalicylicacid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[(p-azidophenyl)dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. Patent Nos.3,969,287;3,691,016;4,195,128; 4,247,642; 4,229,537; and 4,330,440 are employed for protein immobilization.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman &Co., San Francisco, pp. 79-86 (1983)], acetylation of the N-terminal amine, and amidation of any C-terminal carboxyl group. The modified forms of the residues fall within the scope of the present invention.

Another type of covalent modification of the RTD polypeptide included within the scope of this invention comprises altering the native glycosylation pattern of the polypeptide. "Altering the native glycosylation pattern" is intended for purposes herein to mean deleting one or more carbohydrate moieties found in native sequence RTD, and/or adding one or more glycosylation sites that are not present in the native sequence RTD.

Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosy lation site. O-linkled glycosy lation refers to the attachment of one of the sugars N-aceylgalactosamine,galactose, or xylose to a hydroxylaminoacid, most commonly serine or threonine, although 5-hydroxyproline or 5-hydroxylysine may also be used.

Addition of glycosylation sites to the RTD polypeptide may be accomplished by altering the amino acid sequence such that it contains one or more of the above-described tripeptide sequences (for N-linked glycosylation sites). The alteration may also be made by the addition of, or substitution by, one or more serine or threonine residues to the native sequence RTD (for O-linked glycosylation sites). The RTD amino acid sequence may optionally be altered through changes at the DNA level, particularly by mutating the DNA encoding the RTD polypeptide at preselected bases such that codons are generated that will translate into the desired amino acids. The DNA mutation(s) may be made using methods described above and in U.S. Pat. No. 5,364,934, supra.

Another means of increasing the number of carbohydrate moieties on the RTD polypeptide is by chemical or enzymatic coupling of glycosides to the polypeptide. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published 11 September 1987, and in Aplin and Wriston, CRC Crit. Rev.Biochem., pp. 259-306 (1981).

Removal of carbohydrate moieties present on the RTD polypeptide may be accomplishedchemically or enzymatically or by mutational substitution of codons encoding for amino acid residues that serve as targets for glycosylation. For instance, chemical deglycosylation by exposing the polypeptide to the compound trifluoromethanesulfonic acid, or an equivalent compound can result in the cleavage of most or all sugars except the linking sugar (N-acetylglucosamineor N-acetylgalactosamine),while leaving the polypeptide intact. Chemical deglycosylation is described by Hakimuddin, et al., Arch, Biochem, Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidasesas described by Thotakura et al., Meth. Enzymol., 138:350 (1987).

Glycosylat ion at potential glycosylation sites may be prevented by the use of the compound tunicamycin as described by Duskin et al., J. Biol. Chem., 257:3105 (1982). Tunicamycin blocks the formation of protein-N-glycoside linkages.

Another type of covalent modification of RTD comprises linking the RTD polypeptide to one of a variety of nonproteinaceouspolymers,*e.g*., polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

### 8. RTD Chimeras

The present invention also provides chimeric molecules comprising RTD fused to another, heterologous polypeptide or amino acid sequence.

In one embodiment, the chimeric molecule comprises a fusion of the RTD with a tag polypeptide which provides an epitope to which an anti-tag antibody can selectively bind. The epitope tag is generally placed at the amino- or carboxyl- terminus of the RTD. The presence of such epitope-tagged forms of the RTD can be detected using an antibody against the tag polypeptide. Also, provision of the epitope tag enables the RTD to be readily purified by affinity purification using an anti-tag antibody or another type of affinity matrix that binds to the epitope tag.

Various tag polypeptides and their respective antibodies are well known in the art. Examples include the poly his tag; flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194(1992)]; an α-tubulin epitope peptide [Skinner et al., J.Biol.Chem., 266:15163-15166 (1991)]; and the T7 gene 10 protein peptide tag [Lutz-Freyermuth et al., Proc. Natl. Acad. Sci. USA, 87:6393.6397 (1990)]. Once the tag polypeptide has been selected, an antibody thereto can be generated using the techniques disclosed herein.

Generally, epitope-taggedRTD may be constructed and produced according to the methods described above. RTD-tag polypeptide fusions are preferably constructed by fusing the cDNA sequence encoding the RTD portion in-frameto the tag polypeptide DNA sequence and expressing the resultant DNA fusion construct in appropriate host cells. Ordinarily, when preparing the RTD-tag polypeptide chimeras of the present invention, nucleic acid encoding the RTD will be fused at its 3' end to nucleic acid encoding the N-terminus of the tag polypeptide, however 5' fusions are also possible. For example, a polyhistidine sequence of about 5 to about 10 histidine residues may be fused at the N- terminus or the C- terminus and used as a purification handle in affinity chromatography.

Epitope-tagged RTD can be purified by affinity chromatography using the anti-tag antibody. The matrix to which the affinity antibody is attached may include, for instance, agarose, controlled pore glass or poly(styrenedivinyl)benzene. The epitope-tagged RTD can then be eluted from the affinity column using techniques known in the art.

In another embodiment, the chimeric molecule comprises an RTD polypeptide fused to an immunoglobulin sequence. The chimeric-molecule may also comprise a particular domain sequence of RTD, such as the extracellular domain sequence of native RTD fused to an immunoglobu lin sequence. This includes chimeras in monomeric, homo- or heteromultimeric, and particularly homo- or heterodimeric, or -tetrameric forms; optionally, the chimeras may be in dimeric forms or homodimeric heavy chain forms. Generally, these assembled immunoglobulins will have known unit structures as represented by the following diagrams:

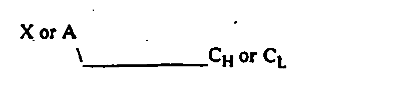

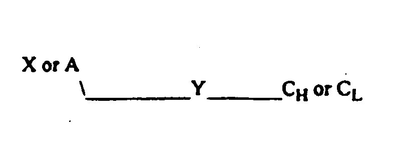

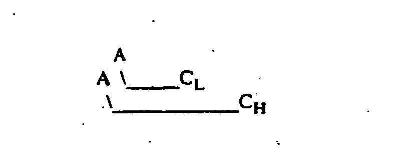

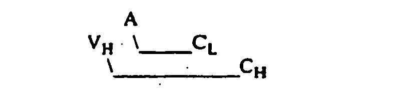

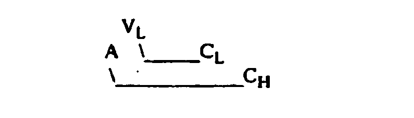

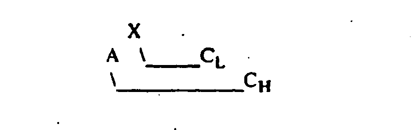

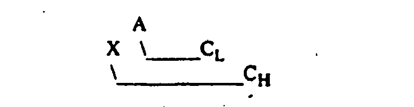

A basic four chain structural unit is the form in which IgG, IgD, and IgE exist. A four chain unit is repeated in the higher molecular weight immunoglobulins; IgM generally exists as a pentamer of basic four-chain units held together by disulfide bonds. IgA globulin, and occasionally IgG globulin, may also exist in a multimeric form in serum. In the case of multimers, each four chain unit may be the same or different.

The following diagrams depict some exemplary monomer, homo- and heterodimer and homo- and heteromultimer structures. These diagrams are merely illustrative,and the chains of the multimers are believed to be disulfide bonded in the same fashion as native immunoglobulins. 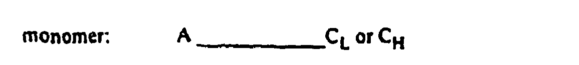 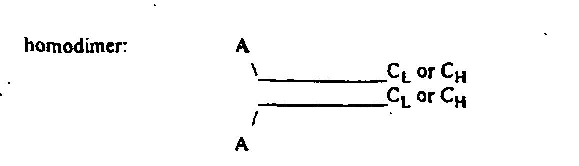 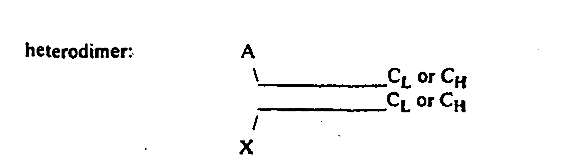 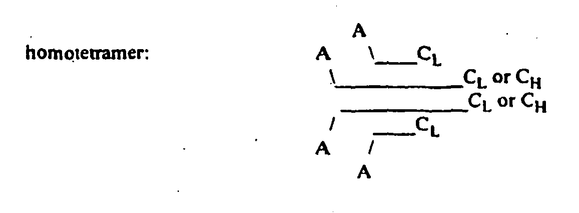 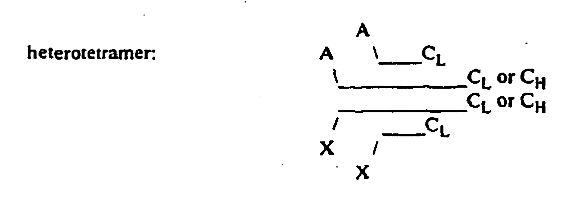 and

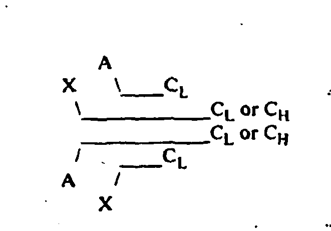

In the foregoing diagrams, "A" means an RTD sequence or a RTD sequence fused to a heterologous sequence; X is an additional agent, which may be the same as A or different, a portion of an immunoglobulin superfamily member such as a variable region or a variable region-tikedomain, including a native or chimeric immunoglobulin variable region, a toxin such a pseudomonas exotoxin or ricin, or a sequence functionally binding to another protein, such as other cytokines (i.e., IL-1, interferon-γ) or cell surface molecules (i.e., NGFR, CD40, OX40, Fas antigen, T2 proteins of Shope and myxoma poxviruses),or a polypeptide therapeutic agent not otherwise normally associated with a constant domain; Y is a linker or another receptor sequence; and V_{L}, V_{H}, C_{L} and C_{H} represent light or heavy chain variable or constant domains of an immunoglobulin. Structures comprising at least one CRD of a RTD sequence as "A" and another cell-surface protein having a repetitive pattern of CRDs (such as TNFR) as "X" are specifically included.

It will be understood that the above diagrams are merely exemplary of the possible structures of the chimeras of the present invention, and do not encompass all possibilities. For example, there might desirably be several different "A"s, "X"s, or "Y"s in any of these constructs. Also, the heavy or light chain constant domains may be originated from the same or different immunoglobulins. All possible permutations of the illustrated and similar structures are all within the scope of the invention herein.

In general, the chimeric molecules can be constructed in a fashion similar to chimeric antibodies in which a variable domain from an antibody of one species is substituted for the variable domain of another species. See, for example, EP 0 125 023; EP 173,494; Munro, Nature, 312 :597 (13 December 1984); Neuberger et al., Nature, 312:604-608 (13 December 1984); Sharon et al., Nature, 309:364.367 (24 May 1984); Morrison et al., Proc. Nat'l. Acad. Sci. USA, 81:6851-6855 (1984); Morrison et al., Science, 229:1202-1207 (1985); Boulianneet al., Nature, 312:643-646 (13 December 1984); Capon et al., Nature, 337:525-531 (1989); Traunecker et al., Nature, 339:68-70 (1989).

Alternatively, the chimeric molecules may be constructed as follows. The DNA including a region encoding the desired sequence, such as a RTD and/or TNFR sequence, is cleaved by a restriction enzyme at or proximal to the 3' end of the DNA encoding the immunoglobulin-like domain(s) and at a point at or near the DNA encoding the N-terminal end of the RTD or TNFR polypeptide (where use of a different leader is contemplated) or at or proximal to the N-terminal coding region for TNFR (where the native signal is employed). This DNA fragment then is readily inserted proximal to DNA encoding an immunoglobulin light or heavy chain constant region and, if necessary, the resulting construct tailored by deletional mutagenesis. Preferably, the Ig is a human immunoglobulin when the chimeric molecule is intended for in vivo therapy for humans. DNA encoding immunoglobulin light or heavy chain constant regions is known or readily available from cDNA libraries or is synthesized. See for example, Adams et al., Biochemistry, 19:2711-2719 (1980); Gough et al., Biochemistry, 19:2702-2710 (1980); Dolby et al., Proc. Natl. Acad. Sci. USA, 77:6027-6031 (1980); Rice et al., Proc. Natl. Acad. Sci., 79:7862-7865 (1982); Falkner et al.. Nature, 298:286-288 (1982); and Morrison et al., Ann.Rev. Immunol., 2:239-256 (1984).

Further details of how to prepare such fusions are found in publications concerning the preparation of immunoadhesins. Immunoadhesins in general, and CD4-Ig fusion molecules specifically are disclosed in WO 89/02922, published 6 April 1989). Molecules comprising the extracellular portion of CD4, the receptor for human immunodeficiencyvirus (HIV), linked to IgG heavy chain constant region are known in the art and have been found to have a markedly longer half-life and lower clearance than the soluble extracellular portion of CD4 [Capon et al., supra; Byrn et al., Nature, 344:667 (1990)]. The construction of specific chimeric TNFR-IgG molecules is also described in Ashkenazi et al. Proc. Natl. Acad. Sci., 88:10535-10539 (1991); Lesslauer et al. [J. Cell. Biochem. Supplement 15F, 1991, p. 115 (P 432)]; and Peppel and Beutler, J. Cell, Biochem. Supplement 15F, 1991, p.118 (P 439)].

### B. Therapeutic and Non-therapeutic Uses for RTD

RTD, as disclosed in the presentspecification,can be employed therapeutically to modulate apoptosis and/or NF-κB activation by Apo-2L or by another ligand that RTD binds to in mammalian cells. This therapy can be accomplished for instance, using *in vivo* or *ex vivo* gene therapy techniques. The RTD chimeric molecules (including the chimeric molecules containing an extracellular domain sequence of RTD) comprising immunoglobulin sequences can also be employed to inhibit Apo-2L activities, for example, apoptosis or NF-κB induction or the activity of another ligand that RTD binds to.

Suitable carriers and their formulations are described in Remington's Pharmaceutical Sciences, 16th ed., 1980, Mack Publishing Co., edited by Oslo et al. Typically, an appropriate amount of a pharmaceutically-acceptable salt is used in the formulation to render the formulation isotonic. Examples of the carrier include buffers such as saline, Ringer's solution and dextrose solution. The pH of the solution is preferably from about 5 to about 8, and more preferably from about 7.4 to about 7.8. It will be apparent to those persons skilled in the art that certain carriers may be more preferable depending upon, for instance, the route of administration.

Administration to a mammal may be accomplished by injection (*e.g*., intravenous, intraperitoneal subcutaneous, intramuscular), or by other methods such as infusion that ensure delivery to the bloodstream in an effective form. Effective dosages and schedules for administration may be determined empirically, and making such determinations is within the skill in the art.

It is contemplated, that other, additional therapies may be administered to the mammal, and such includes but is not limited to, chemotherapy and radiation therapy, immunoadjuvants,cytokines, and antibody-based therapies. Examples include interleukins (*e.g*., IL-1, IL-2, IL-3, IL-6), leukemia inhibitory factor, interferons, TGF-beta, erythropoietin, thrombopoietin, and HER-2 antibody. Other agents may also employed, and such agents include TNF-α, TNF-β (lymphotoxin-α), CD30 ligand, 4-IBB ligand, and Apo-I ligand.

Chemotherapies contemplated by the invention include chemical substances or drugs which are known in the art and are commercially available, such as Doxorubicin, 5-Fluorouracil, Cytosine arabinoside ("Ara-C"), Cyclophosphamide, Thiotepa, Busulfan, Cytoxin, Taxol, Methotrexate, Cisplatin, Melphalan, Vinblastine and Carboplatin. Preparation and dosing schedules for such chemotherapy may be used according to manufacturers'instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapy is preferably administered in a pharmaceutically-acceptable carrier, such as those described above.

The RTD of the invention also has utility in non-therapeutic applications. Nucleic acid sequences encoding the RTD may be used as a diagnostic for tissue-specifictyping. For example, procedures like *in situ* hybridization, Northern and Southern blotting, and PCR analysis may be used to determine whether DNA and/or RNA encoding RTD is present in the cell type(s) being evaluated. RTD nucleic acid will also be useful for the preparation of RTD by the recombinant techniques described herein.

The isolated RTD may be used in quantitative diagnostic assays as a control against which samples containing unknown quantities of RTD may be prepared. RTD preparations are also useful in generating antibodies, as standards in assays for RTD (*e.g.*, by labeling RTD for use as a standard in a radioimmunoassay, radioreceptor assay, or enzyme-linked immunoassay), in affinity purification techniques, and in competitive-type receptor binding assays when labeled with, for instance, radioiodine, enzymes, or fluorophores.

Isolated, native forms of RTD, such as described in the Examples, may be employed to identify alternate forms of RTD; for example, forms that possess cytoplasmic domain(s) which may be involved in signaling pathway(s). Modified forms of the RTD, such as the RTD-IgG chimeric molecules (immunoadhesins) described above, can be used as immunogens in producing anti-RTD antibodies.

Nucleic acids which encode RTD or its modified forms can also be used to generate either transgenic animals or "knock out" animals which, in turn, are useful in the development and screening of therapeutically useful reagents. A transgenic animal (e.g., a mouse or rat) is an animal having cells that contain a transgeno, which transgene was introduced into the animal or an ancestor of the animal at a prenatal, e.g., an embryonic stage. A transgene is a DNA which is integrates into the genome of a cell from which a transgenic animal develops. In one embodiment, cDNA encoding RTD or an appropriate sequence thereof (such as RTD-IgG) can be used to clone genomic DNA encoding RTD in accordance with established techniques and the genomic sequences used to generate transgenic animals that contain cells which express DNA encoding RTD. Methods for generating transgenic animals, particularly animals such as mice or rats, have become conventional in the art and are described, for example, in U.S. Patent Nos. 4,736,866 and 4,870,009- Typically, particular cells would be targeted for RTD transgene incorporation with tissue-specific enhancers. Transgenic animals that include a copy of a transgene encoding RTD introduced into the germ line of the animal at an embryonic stage can be used to examine the effect of increased expression of DNA encoding RTD. Such animals can be used as tester animals for reagents thought to confer protection from, for example, pathological conditions associated with excessive apoptosis. In such experiments, an animal is treated with the reagent and a reduced incidence of the pathological condition, compared to untreated animals bearing the transgene, would indicate a potential therapeutic intervention for the pathological condition. Transgenic animals that carry a soluble form of RTD such as the RTD ECD or an immunoglobulin chimera of such form could be constructedto test the effect of chronic neutralization of Apo-2L, a ligand of RTD.

Alternatively, non-human homologues of RTD can be used to construct a RTD "knock out" animal which has a defective or altered gene encoding RTD as a result of homologous recombination between the endogenous gene encoding RTD and altered genomic DNA encoding RTD introduced into an embryonic tell of the animal. For example, cDNA encoding RTD can be used to clone genomic DNA encoding RTD In accordance with established techniques. A portion of the genomic DNA encoding RTD can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915(1992)]. The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson,ed. (IRL, Oxford, 1987), pp.1113.152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the RTD polypeptide, including for example, development of tumors.

### C. Anti-RTD Antibody Preparation

The present invention further provides anti-RTD antibodies. Antibodies against RTD may be prepared as follows. Exemplary antibodies include polyclonal, monoclonal, humanized, bispecific, and heteroconjugate antibodies.

### 1. Polyclonal Antibodies

The RTD antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the RTD polypeptide or a fusion protein thereof. An example of a suitable immunizing agent is a RTD-IgG fusion protein or chimeric molecule (including a RTD ECD-IgG fusion protein). Cells expressing RTD at their surface may also be employed. It may be useful to conjugate the immunizing agent to a protein known to be immunogenic in the mammal being immunized. Examples of such immunogenic proteins which may be employed include but are not limited to keyhole limpet hemocyanin, serum albumin, bovine thyroglobulin,and soybean trypsin inhibitor. An aggregating agent such as alum may also be employed to enhance the mammal's immune response. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation. The mammal can then be bled, and the serum assayed for antibody titer. If desired, the mammal can be boosted until the antibody titer increases or plateaus.

### 2. Monoclonal Antibodies

The RTD antibodies may, alternatively, be monoclonal antibodies. Monoclonal antibodies may be prepared using hybridoma methods, such as those described by Kohler and Milstein, supra. In as hybridoma method, a mouse, hamster, or other appropriate host animal, is typically immunized (such as described above) with an immunizing agent to elicit lymphocytes that produce or are capable of producing antibodies that will specifically bind to the immunizing agent. Alternatively, the lymphocytes may be immunized *in vitro.*

The immunizing agent will typically include the RTD polypeptide or a fusion protein thereof. An example of a suitable immunizing agent is a RTD-IgG fusion protein or chimeric molecule. Cells expressing RTD at their surface may also be employed. Generally,either peripheral blood lymphocytes("PBLs") are used if cells,of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies; Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitableculture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:300 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against RTD. Preferably, the binding specificity of monoclonal antibodies produced by the hybridomacells is determined by immunoprecipitation or by an *in vitro* binding assay, such as radioimmunoassay(RIA) or enzyme-linked immunoabsorbentassay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinantDNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodiesmay be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published 12/22/94 and U.S. Patent No. 4,342,566. Papain digestion of antibodies typically produces two identical antigen binding fragments, called Fab fragments, each with a single antigen binding site, and a residual Fc fragment. Pepsin treatment yields an F(ab')₂ fragment that has two antigen combining sites and is still capable of cross-linking antigen.

The Fab fragments produced in the antibody digestion also contain the constant domains of the light chain and the first constant domain (CH₁) of the heavy chain. Fab' fragments differ from Fab fragments by the addition of a few residues at the carboxy terminus of the heavy chain CH₁ domain including one or more cysteines from the antibody hinge region. Fab'-SH is the designation herein for Fab' in which the cysteine residue(s) of the constant domains bear a free thiol group. F(ab')₂ antibody fragments originally were producedas pairs of Fab' fragments which have hinge cysteines between them. Other chemical couplings of antibody fragments are also known.

### 3. Humanized Antibodies

The RTD antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are thoseof a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature. 332:323.329 (1988); and Presta, Curr. Op. Struct. Biol, 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323.327 (1988); Verhoeyen et al., Science, 232: 1534-1536 (1988)], by substituting rodent CDRs or CDR sequences for the corresponding sequences of a human antibody. Accordingly, such "humanized" antibodies are chimeric antibodies(U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important in orderto reduce antigenicity. According to the "best-fit" method, the sequence of the variable domain of a rodent antibody is screened against the entire library of known human variable domain sequences. The human sequence which is closest to that of the rodent is then accepted as the human framework (FR) for the humanized antibody [Sims et al., J. Immunol., 151:2296 (1993); Chothia and Lesk, J. Mol Biol., 196:901 (1987)]. Another method uses a particular framework derived from the consensus sequence of all human antibodies of a particular subgroup of light or heavy chains. The same framework may be used for several different humanized antibodies [Carter et al., Proc. Natl. Acad. Sci. USA, 89:4285 (1992); Presta et al., J. Immunol., 151: 2623 (1993)].

It is further important that antibodies be humanized with retention of high affinity for the antigen and other favorable biological properties. To achieve this goal, according to a preferred method, humanized antibodies are preparedly a process of analysis of the parental sequences and various conceptual humanized products using three dimensional models of the parental and humanized sequences. Three dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. In general, the CDR residues are directly and most substantially involved in influencing antigen binding [see, WO 94/04679 published 3 March 1994].

Transgenic animals (e.g., mice) that are capable, upon immunization, of producing a full repertoire of human antibodies in the absence of endogenous immunoglobulin production can be employed. For example, it has been described that the homozygous deletion of the antibody heavy chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge [see, e:g., Jakobovits et al., Proc, Natl. Acad. Sci. USA, 90:2551-255 (1993); Jakobovits et al., Nature, 362:255-258 (1993); Bruggermann et al., Year in Immuno., 7:33 (1993)]. Human antibodies can also be produced in phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. immunol., 147(1):86-95 (1991)].

### 4. Bispecific Antibodies

Bispecific antibodies are monoclonal, preferably human or humanized, antibodies that have binding specificities for at least two different antigens. In the present case, one of the binding specificities is for the RTD, the other one is for any other antigen, and preferably for a cell-surface protein or receptor or receptor subunit.

Methods for making bispecific antibodies are known in the art. Traditionally, the recombinant production of bispecificantibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture often differentantibody molecules, of which on ly one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

According to a different and more preferred approach, antibody variable domains with the desired binding specificities (antibody-antigen combining sites) are fused to immunoglobulin constant domain sequences. The fusion preferably is with an immunoglobulinheavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance. In a preferred embodiment of this approach, the bispecific antibodies are composed of a hybrid immunoglobulin heavy chain with a first binding specificity in one arm, and a hybrid immunoglobulin, heavy-chain/light-chainpair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690 published 3 March 1994. For further details of generating bispecific antibodies see, for example, Suresh et al., Methods in Enzymology, 121:210 (1986).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [US Patent No. 4,676,980], and for treatment of HIV infection [WO 91/00360; WO 92/2003-73; BP 03089]. It is contemplatedthat the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include Iminothiolate and methyl-4-mercaptobutyrimidate and those disclosed, for example, in U.S. Pat. No. 4,676,980.

### D. Therapeutic and Non-therapeutic Uses for RTD Antibodies

The RTD antibodies of the invention have therapeutic utility. For example, antagonistic antibodies may be used to sensitize cells to Apo-2 ligand induced apoptosis.

RTD antibodies may further be used in diagnostic assays for RTD, e.g., detecting its expression in specific cells, tissues, or serum. Various diagnostic assay techniques known in the art may be used, such as competitivebinding assays, direct or indirect sandwich assays and immunoprecipitation assays conducted in either heterogeneousor homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature. 144:945 (1962); David et al., Biochemistry, 13:1014 (1974); Pain et al., J. Immunol, Meth., 40:219 (1981); and Nygren, J. Histochem and Cytochem., 30:407 (1982).

RTD antibodies also are useful for the affinity purification of RTD from recombinant cell culture or natural sources, In this process, the antibodies against RTD are immobilized on a suitable support, such a Sephadex resin or filter paper, using methods well known in the art. The immobilized antibody then is contacted with a sample containing the RTD to be purified, and thereafter the support is washed with a suitable solvent that will remove substantially all the material in the sample except the RTD, which is bound to the immobilized antibody. Finally, the support is washed with another suitable solvent that will release the RTD from the antibody.

The following examples are offered for illustrative purposes only, and are not intended to limit the scope of the present invention in any way.

All patent and literature references cited in the present specification are hereby incorporated by reference in their entirety.

### EXAMPLES

All restriction enzymes referred to in the examples were purchased from New England Biolabs and used according to manufacturer's instructions. All other commercially available reagents referred to in the examples were used according to manufacturer's instructions unless otherwise indicated. The source of those cells identified in the following examples, and throughout the specification, by ATCC accession numbers is the American Type Culture Collection, Manassas, Virginia.

### EXAMPLE 1

### Isolation of cDNA clones Encoding Human RTD

A synthetic probe based on the sequence encoding the DcR1 ECD [Sheridan et al., supra] and having the following sequence:
CATAAAAGTTCCTGCACCATGACCAGAGACACAGTGTGTCAGTGTAAAGA (SEQ ID NO:3)
was used to screen a human fetal lung cDNA library. To prepare the cDNA library, mRNA was isolated from human fetal lung tissue using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System): In this procedure, the double stranded cDNA was sized to greater than 1000 bp and the SalI/NotI linkered cDNA was cloned into XhoI/NotI cleaved vector, pRK5D is a cloning vector that has an sp6 transcription initiation site followed by an SfiI restriction enzyme site preceding the XhoI/NotI cDNA cloning sites.

Two full length clones were identified (DNA35663 and DNA35664) that contained a single open reading frame with an apparent translational initiation site at nucleotidepositions 157-159 [Kozak et al., supra] and ending at the stop codon found at nucleotide positions 1315-1317 (Fig. 1A; SEQ ID NO:2). There is a single base difference between the two clones at nucleotide position 1085 (either a C or T) (Fig. 1A), resulting in a serine codon (TCG) (clone DNA35663) or a leucine codon (TTG) (clone DNA35664) at amino acid position 310 (Fig. 1A). These clones are referred to as pRKS-35663 and pRKS-35664 and deposited as ATCC Nos. 209201 and 209202, respectively.

The predicted polypeptide precursor is 386 amino acids long and has a calculated molecular weight of approximately 41.8 kDa. Sequence analysis indicated a N-terminal signal peptide (amino acids 1-55), followed by an ECD (amino acids 56-212), transmembrane domain (amino acids 213-232) and intracellular region (amino acids 233-386). (Figure 1A). The signal peptide cleavage site was confirmed by N-terminal protein sequencing of an RTD ECD immunoadhesin(not shown). This structure suggests that RTD is a type I transmembrane protein. RTD contains 3 potential N-linked glycosylation sites, at amino acid positions 127, 171 and 182. (Fig. 1A) The RTD polypeptides are obtained or obtainable by expressing the polypeptide encoded by the cDNA insert of the vectors deposited as ATCC 209201 or ATCC 209202.

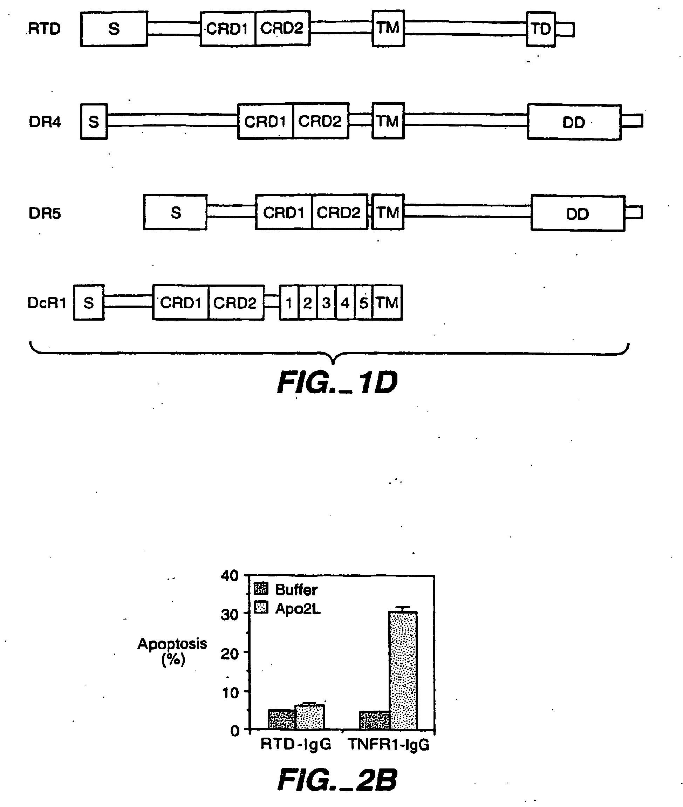

TNF receptor family proteins are typically characterized by the presence of multiple (usually four) cysteine-rich domains in their extracellularregions- each cysteine-richdomain being approximately 45 amino acids long and containing approximately 6, regularly spaced, cysteine residues. Based on the crystal structure of the type 1 TNF receptor, the cysteines in each domain typically form three disulfide bonds in which usually cysteines 1 and 2, 3 and 5, and 4 and 6 are paired together. Like DR4, DR5, and DcR1, RTD contains two extracellular cysteine-rich pseudorepeats (Fig. 1D), whereas other identified mammalian TNFR family members contain three or more such domains [Smith et al., Cell, 76:959 (1994)].

Based on an alignment analysis of the ECD sequence shown in Figure 1B (SEQ ID NO:1), RTD shows more sequence identity to the ECD of DR4 (55%), DR5 (56%), or DcR1 (67%) than to other apoptosis-linked receptors, such as TNFR1 (26%). Fas/Apo-1 (27%) or DR3 (19%). The predicted intracellular sequence of RTD also shows more homology to the corresponding region of DR4 (60%) or DR5 (49%) as compared to TNFR1 (18%), Fas (14%) or DR3 (10%). (Fig. 1C) The intracellular region of RTD is about 50 residues shorter than the intracellular regions identified for DR4 or DR5. It is presently believed that RTD may contain an truncated death domain (amino acids 340-364; Fig. 1D), which corresponds to the carboxy-terminal portion of the death domain sequences of DR4 and DR5. Five out of six amino acids that are essential for signaling by TNFR1 [Tartaglia et al., supra] and that are conserved or semi-conserved in DR4 and DR5, are absent in RTD. (Figure IC).

### EXAMPLE 2

### A. Expression of RTD ECD as an Immunoadhesin

A RTD ECD immunoadhesin was constructed by fusing a cDNA sequence encoding the extracellular region of RTD (amino acids 1-212; see Fig. 1A) to a cDNA encoding the hinge, CH2, and CH3 regions of human IgG1, as described in Ashkenazi et al., supra. Immunoadhesins based on the extracellular region of DR5 [Sheridan et al., supra; Pan et al., supra] or TNFR1 [Ashkenazi et al., supra] were similarly constructed. The immunoadhesinswere expressed as recombinant proteins by transfecting Sf9 cells (ATCC CRL 1711) and purified by protein A affinity chromatography.

### B. Immunoprecipitation Assay Showing

### Binding Interaction between RTD ECD and Apo-2 ligand

The RTD, DR5 or TNFR1 immunoadhesin(2.5 µg) was incubated with ¹²⁵I-labeled soluble Apo-2 ligand [Pitti et al., supra] (1 ng, specific activity 10.7 µCi/µg) in the absence or presence of 1 µg unlabeled Apo-2 ligand for 1 hour at room temperature. Complexes were precipitated by protein A sepharose, and resolved by electrophoresis on a 4-20% gradient SDS polyacrylamide gel (Novex) under reducing conditions. The gel was dried and subjected to phosphorimager analysis on a BAS2000 system (Fuji).

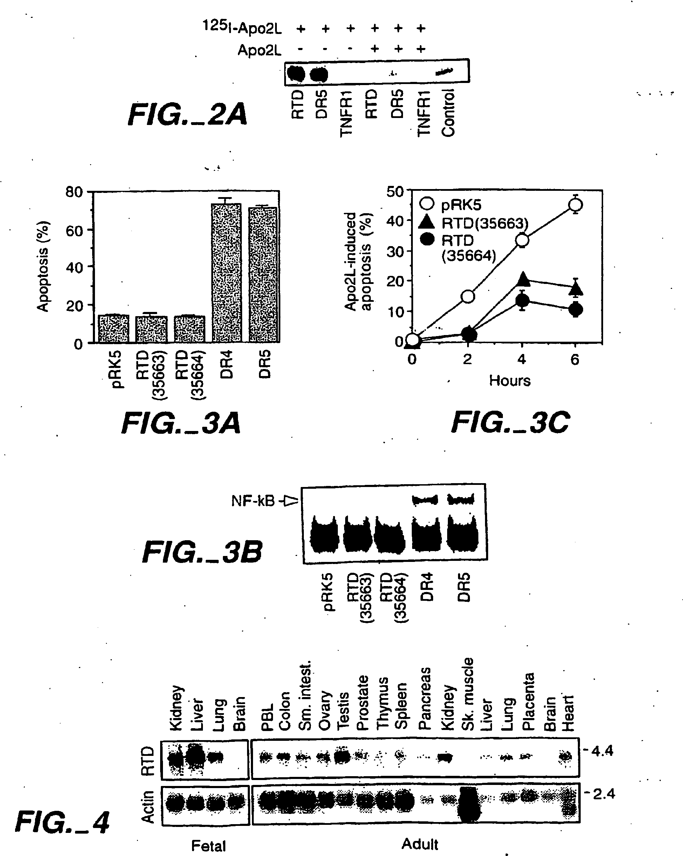

The results are shown in Figure 2A. Both the RTD and DR5 immunoadhesins, but not the TNFR1 immunoadhesin, co-precipitated the labeled Apo-2 ligand. This co-precipitation was blocked by excess unlabeled Apo-2 ligand. The binding interaction was further analyzed on a BIACORE^{™} instrument. BIACORE^{™} analysis demonstrated that the RTD immunoadhesin bound to Apo-2 ligand, but not to other apoptosis-inducing family members, namely, TNF-alpha, lymphotoxin-alpha or Fas ligand (data not shown). These results show that the extracellular region of RTD binds specifically to Apo-2 ligand, supporting the belief that RTD is a receptor for Apo-2 ligand

### EXAMPLE 3

### Inhibition of Apo-2 ligand Function by RTD ECD

HeLa S3 cells (ATCC CCL 2.2) were incubated with PBS buffer or Apo-2 ligand (Pitti et al., supra; 125 ng/ml) in the presence of RTD or TNFR1 immunoadhesins(described in Example 2 above; 10 µg/ml) for 5 hours, and analyzed for apoptosis by annexin V binding as described in Marsters et al., supra. The data, shown in Figure 2B, are the means ± SE of triplicate determinations.

The RTD immunoadhesin, but not the TNFR1 immunoadhesin, blocked Apo-2 ligand's ability to induce apoptosis in HeLa cells (Figure 2B), supporting further the ability of the RTD ECD to bind to Apo-2 ligand, and demonstrating that RTD immunoadhesin is capable of neutralizing Apo-2 ligand.

### EXAMPLE 4

### Inhibition of Apo-2 ligand Function by Full-length RTD

Because death domains can function as oligomerization interfaces, overexpression of receptors that contain such domains can lead to activation of signaling in the absence of ligand [see, Nagata, Cell, 88:355-365 (1997)]. It has been reported that overexpression of DR4 or DR5 can lead to activation of apoptosis and of NF-κB [Sheridan et al., supra; Pan et al., supra]. To investigatewhether RTD can activate apoptosis, HeLa S3 cells were co-transfected with a pRK5-based expression plasmid encoding full-length RTD, along with a plasmid encoding human CD4 as a marker for transfection.

Human HeLa S3 cells (1 x 10⁶ per assay) were transfected by electroporation with pRK5 [Schall et al., Cell, 61:361-370 (1990); Suva, Science, 237:893-896 (1987)], or with pRK5-based plasmids encoding RTD (clone DNA35663 or clone DNA35664), DR4 or DR5 (16 µg), along with pRK5 encoding CD4 (4 µg) as a transfection marker. The level of apoptosis in CD4-expressing cells was assessed 24 hours later, by FACS analysis of annexin V binding, as described in Marsters et al., supra.

As shown in Figure 3A (data represented are means ± SE of triplicate determinations), the RTD-transfected cells showed no difference in the level of apoptosis as compared to pRK5-transfected (control) cells, whereas cells transfected by DR4 or DR5 showed a marked increase in apoptosis.

In another experiment, human 293 cells (ATCC CRL 1573) (5 x 10⁶ per assay) were transfected in 10 cm plates by calcium phosphate precipitation with pRK5 or pRK5-based plasmids encoding RTD (clone DNA35663 or clone DNA35664) or DR5 (20 µg). The cells were analyzed 24 hours later for NF-κB activation by an electrophoretic mobility shift assay, as described by Marsters et al., supra. The results, shown in Figure 3B, reveal that transfectionof 293 cells by RTD did not cause an increase in NF-κB activity, whereas transfection by DR5 caused NF-κB activation. Thus, unlike DR4 and DR5, RTD does not appear to signal apoptosisor NF-κB activation upon overexpression. This suggests that the truncated death domain of RTD is not able to trigger such responses.

In another experiment, 293 cells (1 x 10⁶) were transfected in 6 cm plates by pRK5 or pPK5-based plasmids encoding RTD (clone DNA35663 or clone DNA35664) (4 µg), along with pRK5 encoding green fluorescent protein (GFP; available from Clontech) (1 µg). The cells were treated 24 hours later with Apo-2 ligand (Pitti et al., supra; 0.5 µg/ml), stained with Hoechst 33342 dye (10 µg/ml), and double positive cells were scored for apoptotic morphology under a fluorescence microscope (Leica) equipped with Hoffmann optics.

The results, shown as means ± SE of triplicate determinations, are illustrated in Figure 3C. Cells transfected by either one of the RTD cDNA clones were significantly less sensitive to Apo-2 ligand-induced apoptosis. Similar results were obtained with HeLa cells (data not shown). These results suggest that RTD does not signal cell death and demonstrate that RTD can inhibit Apo-2 ligand function when it is expressed at high levels.

### EXAMPLE 5

### Northern Blot Analysis

Expression of RTD mRNA in human tissues was examined by Northern blot analysis. Human RNA blots were hybridized to a 200 bp ³²P-labelled DNA probe based on the 3' untranslated region of the RTD. The probe was generated by PCR with the following oligonucleotide primers:
CTTCAGGAAACCAGAGCTTCCCTC (SEQ ID NO:4); TTCTCCCGTTTGCTTATCACACGC (SEQ ID NO:5). Probes specific for beta-actin were used as controls. Human fetal RNA blot MTN (Clontech) and human adult RNA blot MTN-It (Clontech) were incubated with the DNA probes. Blots were incubated with the probes in hybridization buffer (5X SSPE; 2X Denhardt's solution; 100 mg/mL denatured sheared salmon sperm DNA; 50% formamide; 2% SDS) for 60 hours at 42°C. The blots were washed several times in 2X SSC; 0.05% SDS for 1 hour at room temperature, followed by a 30 minute wash in 0.1X SSC; 0.1% SDS at 50°C. The blots were developed after overnight exposure by phosphorimager analysis (Fuji).

As shown in Fig. 4, a single RTD mRNA transcript of about 4 kb was detected. This transcript was expressed in fetal kidney, liver and lung, and in multiple adult tissues, particularly in testis and kidney. This mRNA expression pattern differs from that of DR4, DR5 and DcR1. DR4 and DcR1 are particularly abundant in peripheral blood leukocytes and spleen, and DR5 is most abundant in ovary, liver and lung.

### EXAMPLE 6

### Chromosomal Localization of the RTD, DR5, DR4 and DcR1 genes

Chromosomal localization of these human genes was examined by radiation hybrid (RH) panel analysis. RH mapping was performed by PCR using a human-mouse cell radiation hybrid panel (Research Genetics) and primers based on the coding region of the DR5 cDNA [Gelb et al., Hum, Genet., 98: 141 (1996)]. Analysis of the PCR data using the Stanford Human Genome Center Database and the Whitehead Institute for Biomedical Research/MITCenter for Genome Research indicates that DR5 is linked to the marker D8S481, with an LOD of 11.05; D8S481 is linked in turn to D8S2055, which maps to human chromosome 8p21. A similar analysis of DR4 showed that DR4 is linked to the marker D8S2127 (with an LOD of 13.00), which maps also to human chromosome 8p21. Analysis of DcR1 using radiation hybrid panel examination showed that the DcR1 gene is linked to the marker WI-6536, which in turn is linked to D8S298, which maps also to human chromosome 8p21 and is nested between D8S2005 and D8S2127.

Using a primer based on the 3' untranslated region of the RTD cDNA, an analysis revealed that RTD was linked to marker SHGC-33989 (LOD of 7.2). Marker SHGC-33989 is linked to D852055, which maps to human chromosome 8p21. Thus, the human genes for RTD, DR5. DcR1 and DR4, all map to chromosome 8p21.

### Deposit of Material

The following materials have been deposited with the American Type Culture Collection. 10801 University Blvd., Manassas, Virginia, USA (ATCC):

| Material | ATCC Dep. No. | Deposit Date |
|---|---|---|
| pRKS-35663 | 209201 | Aug. 18, 1997 |
| pRK5-35664 | 209202 | Aug. 18, 1997 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc, and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC § 122 and the Commissioner's rules pursuant thereto (including 37 CFR §1.14 with particular reference to 886 OG 638).

The assignee of the present application has agreed that if a culture of the materials on deposit should die or be lost or destroyed when cultivatedunder suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

## Claims

1. Isolated RTD polypeptide having at least 95% amino acid sequence identity with native sequence RTD polypeptide comprising amino acid residues 1 to 386 of Fig. 1A (SEQ ID NO:1), wherein said isolated RTD polypeptide inhibits Apo-2 ligand induced apoptosis in a mammalian cell or binds Apo-2 ligand.

2. The RTD polypeptide of claim 1 comprising amino acid residues 1 to 386 of Fig. 1A (SEQ ID NO:1).

3. The RTD polypeptide of claim 1 comprising amino acid residues 56 to 386 of Fig. 1A (SEQ ID NO:1).

4. Isolated extracellular domain sequence of RTD polypeptide comprising (a) amino acid residues 1 to 212 of Fig. 1A (SEQ ID NO:1); or (b) a fragment of the sequences of (a) which retains the ability to bind Apo-2. ligand.

5. The extracellular domain sequence of claim 4 comprising amino acid residues 56 to 212 of Fig. 1A (SEQ ID NO:1).

6. A chimeric molecules comprising a RTD polypeptide or extracellular domain sequence thereof according to any one of the preceding claims, fused to a heterologous amino acid sequence.

7. The chimeric molecule of claim 6 wherein said heterologous amino acid sequence is an epitope tag sequence.

8. The chimeric molecule of claim 6 wherein said heterologous amino acid sequence is an immunoglobulin sequence.

9. The chimeric molecule of claim 6 wherein said immunoglobulin sequence is an **I**gG**.**

10. An antibody which specifically binds to a RTD polypeptide or extracellular domain sequence thereof as defined in any one of claims 1 to 5.

11. The antibody of claim 10 wherein said antibody is a monoclonal antibody.

12. The antibody of claim 10 which is a chimeric antibody.

13. The antibody of claim 10 which is a humanised antibody.

14. The antibody of claim 10 which is a human antibody.

15. Isolated nucleic acid comprising a nucleotide sequence encoding the RTD polypeptide of claim 1 or the extracellular domain sequence of claim 4 or claim 5.

16. The nucleic acid of claim 15 wherein said nucleotide sequence encodes native sequence RTD polypeptide comprising amino acid residues 1 to 386 of Fig. 1A (SEQ ID NO:1).

17. A vector comprising the nucleic acid of claim 15 or claim 16.

18. The vector of claim 17 operably linked to control sequences recognized by a host cell transformed with the vector.

19. A host cell comprising the vector of claim 17.

20. The host cell of claim 19 which is a mammalian cell.

21. The host cell of claim 19 which is a yeast cell.

22. The host cell of claim 19 which is *E. coli*.

23. A process of using a nucleic acid molecule according to claim 15 or claim 16 to effect production of RTD polypeptide comprising culturing the host cell of any one of claims 19 to 22.

24. A composition comprising RTD polypeptide or extracellular domain sequence thereof as defined in any one of claims 1 to 5 and a carrier.

25. A RTD polypeptide or extracellular domain sequence thereof as defined in any one of claims 1 to 5, or a nucleic acid according to claim 15 or claim 16 for use in therapy.

26. A composition comprising RTD polypeptide or extracellular domain sequence thereof as defined in any one of claims 1 to 5 and a carrier.

27. A method of modulating apoptosis in mammalian cells *in vitro* comprising exposing said cells to RTD polypeptide or extracellular domain sequence thereof an defined in any one of claims 1 to 5.

28. The method of claim 27 wherein said cells are also exposed to Apo-2 ligand.

29. A RTD polypeptide or extracellular domain sequence thereof as defined in any one of claims 1 to 5, or a nucleic acid according to claim 15 or claim 16 for use in therapy.

30. Use of a RTD polypeptide or extracellular domain sequence thereof as defined in any one of claim 1 to 5, or a nucleic acid according to claim 15 or claim 16, in the preparation of a medicament for modulating apoptosis in mammalian cells.

## Patentansprüche

1. Isoliertes RTD-Polypeptid mit zumindest 95 % Aminosäuresequenzidentität mit Nativsequenz-RTD-Polypeptid, umfassend die Aminosäurereste 1 bis 386 aus Fig. 1A (Seq.-ID Nr. 1), worin das isolierte RTD-Polypeptid die Apo-2-Ligand-induzierte Apoptose in einer Säugetierzelle inhibiert oder Apo-2-Ligand bindet.

2. RTD-Polypeptid nach Anspruch 1, umfassend die Aminosäurereste 1 bis 386 aus Fig. 1A (Seq.-ID Nr. 1).

3. RTD-Polypeptid nach Anspruch 1, umfassend die Aminosäurereste 56 bis 386 aus Fig. 1A (Seq.-ID Nr. 1).

4. Isolierte Sequenz der extrazellulären Domäne des RTD-Polypeptids, umfassend (a) die Aminosäurereste 1 bis 212 aus Fig. 1A (Seq.-ID Nr. 1); oder (b) ein Fragment der Sequenz aus (a), das die Fähigkeit, Apo-2-Ligand zu binden, beibehält.

5. Sequenz der extrazellulären Domäne nach Anspruch 4, umfassend die Aminosäurereste 56 bis 212 aus Fig. 1A (Seq.-ID Nr. 1).

6. Chimäres Molekül, umfassend ein RTD-Polypeptid oder eine Sequenz der extrazellulären Domäne davon nach einem der vorangegangenen Ansprüche, fusioniert an eine heterologe Aminosäuresequenz.

7. Chimäres Molekül nach Anspruch 6, worin die heterologe Aminosäuresequenz eine Epitopmarkierungssequenz ist.

8. Chimäres Molekül nach Anspruch 6, worin die heterologe Aminosäuresequenz eine Immunglobulinsequenz ist.

9. Chimäres Molekül nach Anspruch 6, worin die Immunglobulinsequenz ein IgG ist.

10. Antikörper, der spezifisch an ein RTD-Polypeptid oder eine Sequenz der extrazellulären Domäne davon, wie in einem der Ansprüche 1 bis 5 definiert, bindet.

11. Antikörper nach Anspruch 10, worin der Antikörper ein monoklonaler Antikörper ist.

12. Antikörper nach Anspruch 10, der ein chimärer Antikörper ist.

13. Antikörper nach Anspruch 10, der ein humanisierter Antikörper ist.

14. Antikörper nach Anspruch 10, der ein menschlicher Antikörper ist.

15. Isolierte Nucleinsäure, umfassend eine Nucleotidsequenz, die für das RTD-Polypeptid nach Anspruch 1 oder die Sequenz der extrazellulären Domäne nach Anspruch 4 oder Anspruch 5 kodiert.

16. Nucleinsäure nach Anspruch 15, worin die Nucleotidsequenz für Nativsequenz-RTD-Polypeptid, umfassend die Aminosäurereste 1 bis 386 aus Fig. 1A (Seq.-ID Nr. 1), kodiert.

17. Vektor, umfassend die Nucleinsäure nach Anspruch 15 oder Anspruch 16.

18. Vektor nach Anspruch 17, der operabel an Kontrollsequenzen gebunden ist, die von einer mit dem Vektor transformierten Wirtszelle erkannt werden.

19. Wirtszelle, umfassend den Vektor nach Anspruch 17.

20. Wirtszelle nach Anspruch 19, die eine Säugetierzelle ist.

21. Wirtszelle nach Anspruch 19, die eine Hefezelle ist.

22. Wirtszelle nach Anspruch 19, die eine E.-coli-Zelle ist.

23. Verfahren zur Verwendung eines Nucleinsäuremoleküls nach Anspruch 15 oder Anspruch 16, um die Produktion von RTD-Polypeptid zu bewirken, umfassend das Züchten der Wirtszelle nach einem der Ansprüche 19 bis 22.

24. Zusammensetzung, umfassend RTD-Polypeptid oder eine Sequenz der extrazellulären Domäne davon, wie in einem der Ansprüche 1 bis 5 definiert, sowie einen Träger.

25. RTD-Polypeptid oder eine Sequenz der extrazellulären Domäne davon, wie in einem der Ansprüche 1 bis 5 definiert, oder eine Nucleinsäure nach Anspruch 15 oder Anspruch 16 zur therapeutischen Verwendung.

26. Zusammensetzung, umfassend RTD-Polypeptid oder eine Sequenz der extrazellulären Domäne davon, wie in einem der Ansprüche 1 bis 5 definiert, sowie einen Träger.

27. Verfahren zur In-vitro-Modulation von Apoptose in Säugetierzellen, umfassend das Aussetzen der Zellen gegenüber RTD-Polypeptid oder einer Sequenz der extrazellulären Domäne davon, wie in einem der Ansprüche 1 bis 5 definiert.

28. Verfahren nach Anspruch 27, worin die Zellen auch gegenüber Apo-2-Ligand ausgesetzt sind.

29. RTD-Polypeptid oder eine Sequenz der extrazellulären Domäne davon, wie in einem der Ansprüche 1 bis 5 definiert, oder eine Nucleinsäure nach Anspruch 15 oder Anspruch 16 zur therapeutischen Verwendung.

30. Verwendung eines RTD-Polypeptids oder einer Sequenz der extrazellulären Domäne davon, wie in einem der Ansprüche 1 bis 5 definiert, oder einer Nucleinsäure nach Anspruch 15 oder Anspruch 16 zur Herstellung eines Medikaments zur Modulation von Apoptose in Säugetierzellen.

## Revendications

1. Polypeptide RTD isolé ayant une identité de séquence d'aminoacides d'au moins 95 % avec le polypeptide RTD sous forme de la séquence naturelle comprenant les résidus d'aminoacides 1 à 386 de la figure 1A (SEQ ID N° 1), ledit polypeptide RTD isolé inhibant l'apoptose induite par un ligand Apo-2 dans une cellule de mammifère ou se liant au ligand Apo-2.

2. Polypeptide RTD suivant la revendication 1, comprenant les résidus d'aminoacides 1 à 386 de la figure 1A (SEQ ID N° 1).

3. Polypeptide RTD suivant la revendication 1, comprenant les résidus d'aminoacides 56 à 386 de la figure 1A (SEQ ID N° 1).

4. Séquence de domaine extracellulaire isolée du polypeptide RTD, comprenant (a) les résidus d'aminoacides 1 à 212 de la figure 1A (SEQ ID N° 1) ; ou (b) un fragment de la séquence de (a) qui conserve l'aptitude à se lier au ligand Apo-2.

5. Séquence de domaine extracellulaire suivant la revendication 4, comprenant les résidus d'aminoacides 56 à 212 de la figure 1A (SEQ ID N° 1).

6. Molécule chimère comprenant un polypeptide RTD ou sa séquence de domaine extracellulaire suivant l'une quelconque des revendications précédentes, fusionné à une séquence d'aminoacides hétérologue.

7. Molécule chimère suivant la revendication 6, dans laquelle ladite séquence d'aminoacides hétérologue est une séquence de marqueur épitopique.

8. Molécule chimère suivant la revendication 6, dans laquelle ladite séquence d'aminoacides hétérologue est une séquence d'immunoglobuline.

9. Molécule chimère suivant la revendication 6, dans laquelle ladite séquence d'immunoglobuline est une IgG.

10. Anticorps qui se lie spécifiquement à un polypeptide RTD ou à sa séquence de domaine extracellulaire répondant à la définition figurant dans l'une quelconque des revendications 1 à 5.

11. Anticorps suivant la revendication 10, ledit anticorps étant un anticorps monoclonal.

12. Anticorps suivant la revendication 10, qui est un anticorps chimère.

13. Anticorps suivant la revendication 10, qui est un anticorps humanisé.

14. Anticorps suivant la revendication 10, qui est un anticorps humain.

15. Acide nucléique isolé comprenant une séquence de nucléotides codant pour le polypeptide RTD de la revendication 1 ou la séquence de domaine extracellulaire de la revendication 4 ou de la revendication 5.

16. Acide nucléique suivant la revendication 15, dans lequel ladite séquence de nucléotides code pour le polypeptide RTD sous forme de la séquence naturelle comprenant les résidus d'aminoacides 1 à 386 de la figure 1A (SEQ ID N° 1).

17. Vecteur comprenant l'acide nucléique de la revendication 15 ou de la revendication 16.

18. Vecteur suivant la revendication 17, lié de manière fonctionnelle à des séquences de régulation reconnues par une cellule hôte transformée avec le vecteur.

19. Cellule hôte comprenant le vecteur de la revendication 17.

20. Cellule hôte suivant la revendication 19, qui est une cellule de mammifère.

21. Cellule hôte suivant la revendication 19, qui est une cellule de levure.

22. Cellule hôte suivant la revendication 19, qui est une *E*. *coli.*

23. Procédé d'utilisation d'une molécule d'acide nucléique suivant la revendication 15 ou la revendication 16 pour effectuer la production d'un polypeptide RTD, comprenant la culture de la cellule hôte de l'une quelconque des revendications 19 à 22.

24. Composition comprenant un polypeptide RTD ou sa séquence de domaine extracellulaire répondant à la définition figurant dans l'une quelconque des revendications 1 à 5 et un support.

25. Polypeptide RTD ou sa séquence de domaine extracellulaire suivant l'une quelconque des revendications 1 à 5, ou acide nucléique suivant la revendication 15 ou la revendication 16, destiné à être utilisé en thérapie.

26. Composition comprenant un polypeptide RTD ou sa séquence de domaine extracellulaire répondant à la définition figurant dans l'une quelconque des revendications 1 à 5 et un support.

27. Méthode pour moduler l'apoptose dans des cellules de mammifère in vitro, comprenant l'exposition desdites cellules à un polypeptide RTD ou à sa séquence de domaine extracellulaire répondant à la définition figurant dans l'une quelconque des revendications 1 à 5.

28. Méthode suivant la revendication 27, dans laquelle lesdites cellules sont également exposées au ligand Apo-2.

29. Polypeptide RTD ou sa séquence de domaine extracellulaire suivant l'une quelconque des revendications 1 à 7, ou acide nucléique suivant les revendications 15 et 16, destiné à être utilisé en thérapie.

30. Utilisation d'un polypeptide RTD ou de sa séquence de domaine extracellulaire suivant l'une quelconque des revendications 1 à 7, ou d'un acide nucléique suivant les revendications 15 et 16, dans la préparation d'un médicament destiné à moduler l'apoptose dans des cellules de mammifère.


### SEQUENCE LISTING

(1) GENERAL INFORMATION:
   (i) APPLICANT: Genentech, Inc.
   (ii) TITLE OF INVENTION: RTD Receptor
   (iii) NUMBER OF SEQUENCES: 5
   (iv) CORRESPONDENCE ADDRESS:
      (A) ADDRESSEE: Genentech, Inc.
      (B) STREET: 1 DNA Way
      (C) CITY: South San Francisco
      (D) STATE: California
      (E) COUNTRY: USA
      (F) ZIP: 94080
   (v) COMPUTER READABLE FORM:
      (A) MEDIUM TYPE: 3.5 inch, 1.44 Mb floppy disk
      (B) COMPUTER: IBM PC compatible
      (C) OPERATING SYSTEM: PC-DOS/MS-DOS
      (D) SOFTWARE: WinPatin (Genentech)
   (vi) CURRENT APPLICATION DATA:
      (A) APPLICATION NUMBER:
      (B) FILING DATE: 14-Jul-1998
      (C) CLASSIFICATION:
   (vii) PRIOR APPLICATION DATA:
      (A) APPLICATION NUMBER: 08/918874
      (B) FILING DATE: 26-Aug-1997
   (viii) ATTORNEY/AGENT INFORMATION:
      (A) NAME: Marschang, Diane L.
      (B) REGISTRATION NUMBER: 35,600
      (C) REFERENCE/DOCKET NUMBER: P1129R1PCT
   (ix) TELECOMMUNICATION INFORMATION:
      (A) TELEPHONE: 650/225-5416
      (B) TELEFAX: 650/952-9881
(2) INFORMATION FOR SEQ ID NO:1:
   (i) SEQUENCE CHARACTERISTICS:
      (A) LENGTH: 386 amino acids
      (B) TYPE: Amino Acid
      (D) TOPOLOGY: Linear
   (xi) SEQUENCE DESCRIPTION: SEQ ID NO:1:
   Met Gly Leu Trp Gly Gln Ser Val Pro Thr Ala Ser Ser Ala Arg
    1               5                  10                  15
   Ala Gly Arg Tyr Pro Gly Ala Arg Thr Ala Ser Gly Thr Arg Pro
                   20                  25                  30
   Trp Leu Leu Asp Pro Lys Ile Leu Lys Phe Val Val Phe Ile Val
                   35                  40                  45
   Ala Val Leu Leu Pro Val Arg Val Asp Ser Ala Thr Ile Pro Arg
   50 55 60
   Gln Asp Glu Val Pro Gln Gln Thr Val Ala Pro Gln Gln Gln Arg
   65 70 75
   Arg Ser Leu Lys Glu Glu Glu Cys Pro Ala Gly Ser His Arg Ser
   80 85 90
   Glu Tyr Thr Gly Ala Cys Asn Pro Cys Thr Glu Gly Val Asp Tyr
   95 100 105
   Thr Ile Ala Ser Asn Asn Leu Pro Ser Cys Leu Leu Cys Thr Val
   110 115 120
   Cys Lys Ser Gly Gln Thr Asn Lys Ser Ser Cys Thr Thr Thr Arg
   125 130 135
   Asp Thr Val Cys Gln Cys Glu Lys Gly Ser Phe Gln Asp Lys Asn
   140 145 150
   Ser Pro Glu Met Cys Arg Thr Cys Arg Thr Gly Cys Pro Arg Gly
   155 160 165
   Met Val Lys Val Ser Asn Cys Thr Pro Arg Ser Asp Ile Lys Cys
   170 175 180
   Lys Asn Glu Ser Ala Ala Ser Ser Thr Gly Lys Thr Pro Ala Ala
   185 190 195
   Glu Glu Thr Val Thr Thr Ile Leu Gly Met Leu Ala Ser Pro Tyr
   200 205 210
   His Tyr Leu Ile Ile Ile Val Val Leu Val Ile Ile Leu Ala Val
   215 220 225
   Val Val Val Gly Phe Ser Cys Arg Lys Lys Phe Ile Ser Tyr Leu
   230 235 240
   Lys Gly Ile Cys Ser Gly Gly Gly Gly Gly Pro Glu Arg Val His
   245 250 255
   Arg Val Leu Phe Arg Arg Arg Ser Cys Pro Ser Arg Val Pro Gly
   260 265 270
   Ala Glu Asp Asn Ala Arg Asn Glu Thr Leu Ser Asn Arg Tyr Leu
   275 280 285
   Gln Pro Thr Gln Val Ser Glu Gln Glu Ile Gln Gly Gln Glu Leu
   290 295 300
   Ala Glu Leu Thr Gly Val Thr Val Glu Xaa Pro Glu Glu Pro Gln
   305 310 315
   Arg Leu Leu Glu Gln Ala Glu Ala Glu Gly Cys Gln Arg Arg Arg
   320 325 330
   Leu Leu Val Pro Val Asn Asp Ala Asp Ser Ala Asp Ile Ser Thr
   335 340 345
   Leu Leu Asp Ala Ser Ala Thr Leu Glu Glu Gly His Ala Lys Glu
   350 355 360
   Thr Ile Gln Asp Gln Leu Val Gly Ser Glu Lys Leu Phe Tyr Glu
   365 370 375
   Glu Asp Glu Ala Gly Ser Ala Thr Ser Cys Leu
   380 385 386
(2) INFORMATION FOR SEQ ID NO:2:
   (i) SEQUENCE CHARACTERISTICS:
      (A) LENGTH: 2082 base pairs
      (B) TYPE: Nucleic Acid
      (C) STRANDEDNESS: Single
      (D) TOPOLOGY: Linear
   (xi) SEQUENCE DESCRIPTION: SEQ ID NO:2:
   CCAACTGCAC CTCGGTTCTA TCGATTGAAT TCCCCGGGGA TCCTCTAGAG 50
   ATCCCTCGAC CTCGACCCAC GCGTCCGGAA CCTTTGCACG CGCACAAACT 100
   ACGGGGACGA TTTCTGATTG ATTTTTGGCG CTTTCGATCC ACCCTCCTCC 150
   CTTCTC  ATG GGA CTT TGG GGA CAA AGC GTC CCG ACC GCC 189
           Met Gly Leu Trp Gly Gln Ser Val Pro Thr Ala
            1              5                   10
   TCG AGC GCT CGA GCA GGG CGC TAT CCA GGA GCC AGG ACA 228
   Ser Ser Ala Arg Ala Gly Arg Tyr Pro Gly Ala Arg Thr
               15                  20
   GCG TCG GGA ACC AGA CCA TGG CTC CTG GAC CCC AAG ATC 267
   Ala Ser Gly Thr Arg Pro Trp Leu Leu Asp Pro Lys Ile
    25                 30                  35
   CTT AAG TTC GTC GTC TTC ATC GTC GCG GTT CTG CTG CCG 306
   Leu Lys Phe Val Val Phe Ile Val Ala Val Leu Leu Pro
           40                  45                  50
   GTC CGG GTT GAC TCT GCC ACC ATC CCC CGG CAG GAC GAA 345
   Val Arg Val Asp Ser Ala Thr Ile Pro Arg Gln Asp Glu
                       55                  60
   GTT CCC CAG CAG ACA GTG GCC CCA CAG CAA CAG AGG CGC 384
   Val Pro Gln Gln Thr Val Ala Pro Gln Gln Gln Arg Arg
        65                  70                  75
   AGC CTC AAG GAG GAG GAG TGT CCA GCA GGA TCT CAT AGA 423
   Ser Leu Lys Glu Glu Glu Cys Pro Ala Gly Ser His Arg
               80                  85
   TCA GAA TAT ACT GGA GCC TGT AAC CCG TGC ACA GAG GGT 462
   Ser Glu Tyr Thr Gly Ala Cys Asn Pro Cys Thr Glu Gly
    90                    95                  100
   GTG GAT TAC ACC ATT GCT TCC AAC AAT TTG CCT TCT TGC 501
   Val Asp Tyr Thr Ile Ala Ser Asn Asn Leu Pro Ser Cys
           105                 110                 115
   CTG CTA TGT ACA GTT TGT AAA TCA GGT CAA ACA AAT AAA 540
   Leu Leu Cys Thr Val Cys Lys Ser Gly Gln Thr Asn Lys
                   120                 125
   AGT TCC TGT ACC ACG ACC AGA GAC ACC GTG TGT CAG TGT 579
   Ser Ser Cys Thr Thr Thr Arg Asp Thr Val Cys Gln Cys
       130                 135                 140
   GAA AAA GGA AGC TTC CAG GAT AAA AAC TCC CCT GAG ATG 618
   Glu Lys Gly Ser Phe Gln Asp Lys Asn Ser Pro Glu Met
               145                 150
   TGC CGG ACG TGT AGA ACA GGG TGT CCC AGA GGG ATG GTC 657
   Cys Arg Thr Cys Arg Thr Gly Cys Pro Arg Gly Met Val
   155                 160                 165
   AAG GTC AGT AAT TGT ACG CCC CGG AGT GAC ATC AAG TGC 696
   Lys Val Ser Asn Cys Thr Pro Arg Ser Asp Ile Lys Cys
           170                 175                 180
   AAA AAT GAA TCA GCT GCC AGT TCC ACT GGG AAA ACC CCA 735
   Lys Asn Glu Ser Ala Ala Ser Ser Thr Gly Lys Thr Pro
                   185                 190
   GCA GCG GAG GAG ACA GTG ACC ACC ATC CTG GGG ATG CTT 774
   Ala Ala Glu Glu Thr Val Thr Thr Ile Leu Gly Met Leu
       195                 200                 205
   GCC TCT CCC TAT CAC TAC CTT ATC ATC ATA GTG GTT TTA 813
   Ala Ser Pro Tyr His Tyr Leu Ile Ile Ile Val Val Leu
               210                 215
   GTC ATC ATT TTA GCT GTG GTT GTG GTT GGC TTT TCA TGT 852
   Val Ile Ile Leu Ala Val Val Val Val Gly Phe Ser Cys
   220                 225                 230
   CGG AAG AAA TTC ATT TCT TAC CTC AAA GGC ATC TGC TCA 891
   Arg Lys Lys Phe Ile Ser Tyr Leu Lys Gly Ile Cys Ser
           235                 240                 245
   GGT GGT GGA GGA GGT CCC GAA CGT GTG CAC AGA GTC CTT 930
   Gly Gly Gly Gly Gly Pro Glu Arg Val His Arg Val Leu
                   250                 255
   TTC CGG CGG CGT TCA TGT CCT TCA CGA GTT CCT GGG GCG 969
   Phe Arg Arg Arg Ser Cys Pro Ser Arg Val Pro Gly Ala
       260                 265                 270
   GAG GAC AAT GCC CGC AAC GAG ACC CTG AGT AAC AGA TAC 1008
   Glu Asp Asn Ala Arg Asn Glu Thr Leu Ser Asn Arg Tyr
               275                 280
   TTG CAG CCC ACC CAG GTC TCT GAG CAG GAA ATC CAA GGT 1047
   Leu Gln Pro Thr Gln Val Ser Glu Gln Glu Ile Gln Gly
   285                 290                 295
   CAG GAG CTG GCA GAG CTA ACA GGT GTG ACT GTA GAG TYG 1086
   Gln Glu Leu Ala Glu Leu Thr Gly Val Thr Val Glu Xaa
           300                 305                 310
   CCA GAG GAG CCA CAG CGT CTG CTG GAA CAG GCA GAA GCT 1125
   Pro Glu Glu Pro Gln Arg Leu Leu Glu Gln Ala Glu Ala
                   315                 320
   GAA GGG TGT CAG AGG AGG AGG CTG CTG GTT CCA GTG AAT 1164
   Glu Gly Cys Gln Arg Arg Arg Leu Leu Val Pro Val Asn
       325                 330                 335
   GAC GCT GAC TCC GCT GAC ATC AGC ACC TTG CTG GAT GCC 1203
   Asp Ala Asp Ser Ala Asp Ile Ser Thr Leu Leu Asp Ala
               340                 345
   TCG GCA ACA CTG GAA GAA GGA CAT GCA AAG GAA ACA ATT 1242
   Ser Ala Thr Leu Glu Glu Gly His Ala Lys Glu Thr Ile
   350                 355                 360
   CAG GAC CAA CTG GTG GGC TCC GAA AAG CTC TTT TAT GAA 1281
   Gln Asp Gln Leu Val Gly Ser Glu Lys Leu Phe Tyr Glu
           365                 370                 375
   GAA GAT GAG GCA GGC TCT GCT ACG TCC TGC CTG TGAAAG 1320
   Glu Asp Glu Ala Gly Ser Ala Thr Ser Cys Leu
                   380                 385 386
   AATCTCTTCA GGAAACCAGA GCTTCCCTCA TTTACCTTTT CTCCTACAAA 1370
   GGGAAGCAGC CTGGAAGAAA CAGTCCAGTA CTTGACCCAT GCCCCAACAA 1420
   ACTCTACTAT CCAATATGGG GCAGCTTACC AATGGTCCTA GAACTTTGTT 1470
   AACGCACTTG GAGTAATTTT TATGAAATAC TGCGTGTGAT AAGCAAACGG 1520
   GAGAAATTTA TATCAGATTC TTGGCTGCAT AGTTATACGA TTGTGTATTA 1570
   AGGGTCGTTT TAGGCCACAT GCGGTGGCTC ATGCCTGTAA TCCCAGCACT 1620
   TTGATAGGCT GAGGCAGGTG GATTGCTTGA GCTCGGGAGT TTGAGACCAG 1670
   CCTCATCAAC ACAGTGAAAC TCCATCTCAA TTTAAAAAGA AAAAAAGTGG 1720
   TTTTAGGATG TCATTCTTTG CAGTTCTTCA TCATGAGACA AGTCTTTTTT 1770
   TCTGCTTCTT ATATTGCAAG CTCCATCTCT ACTGGTGTGT GCATTTAATG 1820
   ACATCTAACT ACAGATGCCG CACAGCCACA ATGCTTTGCC TTATAGTTTT 1870
   TTAACTTTAG AACGGGATTA TCTTGTTATT ACCTGTATTT TCAGTTTCGG 1920
   ATATTTTTGA CTTAATGATG AGATTATCAA GACGTACCCC TATGCTAAGT 1970
   CATGAGCATA TGGACTTACG AGGGTTCGAC TTAGAGTTTT GAGCTTTAAG 2020
   ATAGGATTAT TGGGGGCTTA CCCCCACCTT AATTAGAAGA AACATTTTAT 2070
   ATTGCTTTAC TA 2082
(2) INFORMATION FOR SEQ ID NO:3:
   (i) SEQUENCE CHARACTERISTICS:
      (A) LENGTH: 50 base pairs
      (B) TYPE: Nucleic Acid
      (C) STRANDEDNESS: Single
      (D) TOPOLOGY: Linear
   (xi) SEQUENCE DESCRIPTION: SEQ ID NO:3:
      CATAAAAGTT CCTGCACCAT GACCAGAGAC ACAGTGTGTC AGTGTAAAGA 50
(2) INFORMATION FOR SEQ ID NO:4:
   (i) SEQUENCE CHARACTERISTICS:
      (A) LENGTH: 24 base pairs
      (B) TYPE: Nucleic Acid
      (C) STRANDEDNESS: Single
      (D) TOPOLOGY: Linear
   (xi) SEQUENCE DESCRIPTION: SEQ ID NO:4:
      CTTCAGGAAA CCAGAGCTTC CCTC 24
(2) INFORMATION FOR SEQ ID NO:5:
   (i) SEQUENCE CHARACTERISTICS:
      (A) LENGTH: 24 base pairs
      (B) TYPE: Nucleic Acid
      (C) STRANDEDNESS: Single
      (D) TOPOLOGY: Linear
   (xi) SEQUENCE DESCRIPTION: SEQ ID NO:5:
      TTCTCCCGTT TGCTTATCAC ACGC 24